# EUROPÄISCHE PATENTSCHRIFT

(11) **EP 3 984 458 B1**
(45) Veröffentlichungstag und Bekanntmachung des Hinweises auf die Patenterteilung: **15.01.2025**
(21) Anmeldenummer: 20201566.5
(22) Anmeldetag: 13.10.2020
(51) Int. Cl.: A61B 6/00, A61B 5/00, A61B 8/00, G06F 3/01, A61B 6/46, A43B 3/34, A43B 3/38, A43B 17/00, A61B 6/10, A43B 3/00, A43B 13/00

(54) **GESTENBASIERTE SIMULTANE ANSTEUERUNG EINER MEDIZINTECHNISCHEN EINRICHTUNG**
GESTURE-BASED SIMULTANEOUS CONTROL OF MEDICAL EQUIPMENT
COMMANDE SIMULTANÉE BASÉE SUR LES GESTES POUR UN DISPOSITIF TECHNIQUE MÉDICAL

(43) Veröffentlichungstag der Anmeldung: 20.04.2022
(73) Patentinhaber: Siemens Healthineers AG, 91301 Forchheim (DE)
(72) Erfinder: Dietz, Volker, 91315 Höchstadt an der Aisch (DE); Dittrich, Steffen, 91058 Erlangen (DE); Jones, Jennifer, 90427 Nürnberg (DE); Leis, Tobias, 84061 Ergoldsbach (DE); Limpert, Milena, 91054 Erlangen (DE); Maass, Nicole, 91336 Heroldsbach (DE); Riess, Steffen, 90610 Winkelhaid (DE); Ruf, Marcel, 91094 Langensendelbach (DE)
(74) Vertreter: Siemens Healthineers Patent Attorneys

(56) Entgegenhaltungen:
- US-A1- 2016 299 570
- US-A1- 2017 103 440
- US-A1- 2017 336 870

## Beschreibung

Die Erfindung betrifft eine Befehlsübermittlungseinrichtung. Weiterhin betrifft die Erfindung ein Befehlsübermittlungssystem. Überdies betrifft die Erfindung ein Befehlsübermittlungsverfahren.

Mit Hilfe moderner bildgebender Verfahren werden in der Medizin häufig zwei- oder dreidimensionale Bilddaten erzeugt, die zur Visualisierung eines abgebildeten Untersuchungsobjekts und darüber hinaus auch für weitere Anwendungen genutzt werden können.

Beispiele für moderne bildgebende medizintechnische Verfahren sind die Computertomographie (CT), die Magnetresonanztomographie, der Einsatz sogenannter PET-Scanner und die Röntgenbildgebung mit C-Bogensystemen.

Um die Sicherheit der Patienten zu gewährleisten, ist bei all diesen Verfahren vorgesehen, dass eine bildgebende medizinische Diagnostik erst dann gestartet werden kann, wenn eine qualifizierte Bedienperson, zum Beispiel eine medizinischtechnische Assistentin (MTA), ein Radiologe oder ein Arzt, mittels einer manuellen Eingabe die Aktivierung des Gerätes anweist. Herkömmliche Mittel zur manuellen Eingabe eines Aktivierungsbefehls in eine medizintechnische Einrichtung sind Schalter, Tasten bzw. Taster und Knöpfe, wobei die Schalter, Taster, Tasten und Knöpfe in der Regel direkt auf oder an der medizintechnischen Einrichtung angeordnet sind. Alternativ können die genannten Aktivierungsmittel auch über eine elektrische Kabelverbindung oder eine elektromagnetische Funkverbindung mit einer medizintechnischen Einrichtung verbunden sein. Darüber hinaus können zusätzliche Schnittstellen zur Interaktion zwischen einer Bedienperson und einer medizintechnischen Einrichtung, wie zum Beispiel ein Drucktaster, ein Touch-Display oder ganz allgemein solche mechanischen oder mechatronischen Einheiten vorhanden sein, die vom Bediener per Hand oder per Fuß betätigt werden können, zum Beispiel ein Fußschalter. Die genannten Eingabeeinheiten sind meist Teil eines Bedienmoduls.

Daneben besteht auch das Problem, die Bedienelemente zu reinigen und zu desinfizieren, um die Übertragung von Krankheiten im klinischen Bereich zu reduzieren. Herkömmliche Bedienmodule liegen meist auf dem Fußboden und können daher leicht verschmutzt werden, mit organischen Stoffen, insbesondere Körperflüssigkeiten, kontaminiert werden und außerdem leicht durch Darüberstolpern beschädigt werden.

Zur Betätigung muss sich eine Bedienperson gewöhnlich in unmittelbarer Nähe eines solchen Bedienmoduls, beispielsweise ein Fußschalter, befinden. Mithin ist die Bedienperson an einen bestimmten Ort gebunden, wenn sie ein bestimmtes medizintechnisches Gerät aktivieren möchte. Ein typisches Bedienmodul zur Aktivierung eines medizintechnischen Geräts ist ein Fußschalter. Allerdings ist mit einem solchen üblichen Fußschalter das Problem verbunden, dass er mindestens eine elektrische Leitung zur Stromversorgung oder Datenübermittlung benötigt, die über den Fußboden eines Untersuchungsraums oder eines Operationsraums verläuft und eine Stolperfalle für das medizinische Personal darstellt.

Ferner werden auch Joysticks eingesetzt, um zum Beispiel C-Bögen zu betätigen. Mit einem Joystick kann man eine wertkontinuierliche Größe einer Bewegung ändern, also beispielsweise eine geringe Auslenkung mit einer geringen Geschwindigkeit ausführen und eine starke Auslenkung mit einer hohen Geschwindigkeit betreiben.

Joysticks werden auch zum Steuern von Bewegungsabläufen in der Automobilbranche, der Luftfahrt sowie der Robotik eingesetzt. Ein solcher Joystick kann zum Beispiel in der Arbeitshöhe einer sitzenden Person angeordnet sein und unterliegt daher keiner so starken Verschmutzung wie eine auf dem Boden befindliche Einheit. Allerdings hat ein Joystick den Nachteil, dass der Benutzer zum Betrieb des Geräts die Hände benutzen muss und daher nicht gleichzeitig die Hände frei hat, um zum Beispiel einen Patienten zu untersuchen oder zu behandeln.

Weiterhin werden zur Aktivierung medizintechnischer Geräte auch andere von Hand oder per Fuß bedienbare Einrichtungen mit Funkanbindung genutzt. Allerdings haben diese Geräte im Falle einer Funkfernbedienung per Hand ebenfalls den Nachteil, dass die Bedienperson nicht die Hände frei hat für weitere Interaktionen.

Auch werden mobile Fußbedieneinrichtungen genutzt, die per Funk mit einem anzusteuernden medizintechnischen System verbunden sind. Mit Fußschaltern werden vorwiegend Schaltvorgänge realisiert, wie zum Beispiel das Einschalten und Ausschalten einer Strahlung oder ein Verfahren eines Patiententischs.

Bei einer solchen Einrichtung bestehen weder Kabel als Stolperfallen noch müssen die Hände für eine Bedienung der mobilen Fußbedieneinrichtung genutzt werden. Liegen allerdings eine Vielzahl von solchen mobilen Fußbedieneinrichtungen auf dem Boden verstreut herum, so stellen in diesem Fall die Fußbedieneinrichtungen selbst Stolperfallen dar. Auch treten bei drahtloser Übertragung die vorstehend beschriebenen Hygieneprobleme auf.

Neben der Betätigung eines elektromechanischen Schaltelements zur Aktivierung oder Deaktivierung eines medizintechnischen Geräts ist es wünschenswert, wenn eine Bedienperson auch weitere Funktionen des medizintechnischen Geräts steuern kann. Beispiele hierfür sind das Scrollen von Bildern bzw. das Durchlaufen einer Sequenz von Bildern, die Veränderung des Bildkontrasts, das Hineinzoomen oder Herauszoomen bei Bilddarstellungen oder das Verändern von anderen für eine Modalität spezifischen Parametern.

Weiterhin ist es wünschenswert, die Bedienung sicherheitsrelevanter Funktionen, durch einen von einem ersten Bedienungskanal unabhängigen Zweitkanal abzusichern. Beispielsweise kann die Bedienung einer Strahlungsauslösung oder einer Bewegung eines Medizintechnikgeräts durch einen Fußschalter zusätzlich durch eine Sprachsteuerung, ein kamerabasiertes Monitoring oder ein HF-basiertes Monitoring einer vorbestimmten Geste bestätigt werden. Gesten, die durch bildliche Erfassung erkannt werden, können zum Beispiel durch Bewegen der Hände oder Finger der Bedienperson sichtbar gemacht werden, wobei die Gesten ein Greifen, Loslassen, Bewegen oder Zoomen bedeuten können. Die Bewegung von Füßen oder Zehen kann zum Beispiel eine Erhöhung einer Geschwindigkeit, eine Verlangsamung, eine Bewegung nach links oder eine Bewegung nach rechts steuern. Eine Augenbewegung nach links, rechts, oben oder unten kann eine Richtungsentscheidung darstellen. Eine Kopfbewegung nach links oder rechts kann ebenfalls für eine Richtungsänderung genutzt werden. Zudem kann bei den durch eine Geste gesteuerten Aktionen eine Rückmeldung durch ein haptisches Signal, zum Beispiel in Form einer Vibration erfolgen, um der Bedienperson das Auslösen einer gewünschten Aktion zu bestätigen.

Durch eine Gestensteuerung kann die Bedienperson simultan ein medizintechnisches Gerät steuern und einen Patienten behandeln. Dies wird dadurch ermöglicht, dass für das Auslösen einer Aktion des medizintechnischen Geräts nicht die Position geändert werden muss, um ein Eingabegerät zu betätigen. Auf diese Weise kann die Bedienperson eine Aktion auslösen, ohne ihre Fokussierung auf eine aktuelle Behandlungstätigkeit zu verlieren. Die Bedienperson kann auch eine Steuerung einer medizintechnischen Einrichtung vornehmen, wenn sie ihre Hände oder Augen für eine Behandlung des Patienten oder eine Betätigung einer anderen medizintechnischen Einrichtung benötigt, indem sie zum Beispiel Befehle per Fußbewegung gibt. Auch kann bei einer solchen simultanen Vorgehensweise medizintechnisches Personal eingespart werden.

US 2017/3366870 offenbart ein Befehlsübermittlungssystem, aufweisend:
- eine erste Befehlsübermittlungseinrichtung, welche
- eine Sensoreinheit zum Erfassen einer Bewegung einer Bedienperson (P),
- eine Fixierungseinheit zum Fixieren der Sensoreinheit und/oder der Befehlsübermittlungseinrichtung an der Bedienperson;
- eine Auswertungseinheit zum Auswerten der erfassten Bewegung der Bedienperson und zum Identifizieren eines Befehls auf Basis der erfassten Bewegung, und
- eine Befehlsübermittlungseinheit zum Übermitteln eines Steuerbefehls auf Basis des identifizierten Befehls an eine zu bedienende medizintechnische Einrichtung aufweist.

Mithin ist es Aufgabe der vorliegenden Erfindung, eine Betätigung und Aktivierung eines medizintechnischen Geräts mit verbesserter Sicherheit gegenüber Unfällen und reduziertem Aufwand für ein Sicherstellen der Hygiene in einem Untersuchungsbereich bereitzustellen, welche auch bei einer simultan dazu durchgeführten Behandlung eines Patienten sicher und komfortabel umsetzbar ist.

Diese Aufgabe wird erfindungsgemäß durch ein Befehlsübermittlungssystem nach Anspruch 1 und ein Befehlsübermittlungsverfahren nach Anspruch 15 gelöst.

Die erfindungsgemäße Befehlsübermittlungseinrichtung weist eine Sensoreinheit zum Erfassen einer körperlichen Bewegung einer Bedienperson auf. Eine solche Sensoreinheit umfasst mindestens einen Sensor. Der Sensor ist dazu eingerichtet, eine Bewegung zu detektieren. Eine solche Bewegung lässt sich zum Beispiel durch eine oder mehrere Beschleunigungsmessungen oder eine Messung einer mechanischen Verformung ermitteln. Die Bewegung kann einem vorab festgelegten Bewegungsmuster folgen, welches zur Erzeugung einer Befehlsinformation eingesetzt wird. Die Sensoreinheit und/oder gar die gesamte Befehlsübermittlungseinrichtung sind durch eine Fixierungseinheit an der Bedienperson befestigt. Dabei soll erwähnt werden, dass die Befehlsübermittlungseinrichtung auch vorzugsweise dazu eingerichtet ist, nicht nur die Sensordaten der internen Sensoreinheit zu verarbeiten, sondern zusätzlich auch Sensordaten von externen Sensoren zu empfangen und diese auszuwerten. Die Fixierungseinheit ermöglicht eine Nutzung der Sensoreinheit, vorzugsweise der Befehlsübermittlungseinrichtung, als eingebettetes System, welches eine Bedienperson stets mit sich führt, ohne dass sie eine Steuereinheit, beispielsweise einen Joystick, festhalten muss.

Teil der erfindungsgemäßen Befehlsübermittlungseinrichtung ist auch eine Auswertungseinheit zum Auswerten der erfassten Bewegung der Bedienperson und zum Identifizieren eines Befehls auf Basis der erfassten Bewegung. Die Auswertungseinheit umfasst vorzugsweise einen sogenannten Ultra-Low-Power Mikrocontroller, welcher mit geringen Energiemengen, beispielsweise durch ein an oder in der Befehlsübermittlungseinrichtung angeordnetes autonomes Energieerzeugungssystem betrieben werden kann.

Die von den Sensoren erfassten Sensordaten, vorzugsweise die durch die Sensoreinheit erfasste Bewegung der Bedienperson, werden von der Auswertungseinheit analysiert. Die Auswertungseinheit klassifiziert die erfasste Bewegung dahingehend, ob es sich um eine beabsichtigte oder eine unbeabsichtigte Bewegung handelt. Eine solche Klassifizierung kann zum Beispiel durch Vergleich der erfassten Bewegung mit einer oder mehreren Referenzbewegungen, welche jeweils einem bestimmten Befehl zugeordnet sind, erfolgen. Die Sensoreinheit umfasst also ein mechatronisches Bauelement, mit dem eine Bewegung einer Bedienperson erfasst werden kann. Ist ein Befehl erkannt bzw. identifiziert, so wird ein Steuerbefehl auf Basis des identifizierten Befehls, beispielsweise der identifizierte Befehl selbst, durch eine Befehlsübermittlungseinheit, die ebenfalls Teil der erfindungsgemäßen Befehlsübermittlungseinrichtung ist, an eine zu bedienende medizintechnische Einrichtung übermittelt.

Die erfindungsgemäße Befehlsübermittlungseinrichtung ist also als mobile Einrichtung in Form eines sogenannten "Smart Wearables" ausgebildet, welches die Bedienperson stets mit sich trägt. Teile oder die gesamte Befehlsübermittlungseinrichtung können als sogenanntes eingebettetes System ausgebildet sein, welches speziell auf eine Ansteuerung eines medizintechnischen Geräts durch Bewegung ausgelegt ist. Vorteilhaft kann eine Bedienperson einer medizintechnischen Einrichtung einen Bedienvorgang durch eine beliebige vorbestimmte Bewegung steuern. Dadurch wird es ermöglicht, parallel mehrere Aufgaben zu erfüllen. Beispielsweise kann die Bedienperson einen Patienten mit ihren Händen untersuchen und gleichzeitig mit einem anderen Körperteil eine medizintechnische Einrichtung, wie zum Beispiel eine bildgebende medizintechnische Einrichtung, steuern. Zudem führt die Bedienperson die Eingabeeinheit in Form der erfindungsgemäßen Befehlsübermittlungseinrichtung stets bei sich, so dass diese niemandem im Weg liegen kann oder zum Staubfänger wird und die Bedienperson nicht danach suchen muss, wenn sie einen Befehl übermitteln will.

Mithin lassen sich auch Hygienebestimmungen in Untersuchungsräumen oder Operationsräumen leichter einhalten, weil die Bedienelemente nicht so leicht verschmutzen können und im besten Fall, wenn jede Person eine eigene Befehlsübermittlungseinrichtung besitzt, jeweils nur von einer einzigen Person genutzt werden. Zudem lässt sich eine Ansteckungsgefahr zwischen einzelnen Bedienpersonen durch eine Schmierinfektion vermeiden, da eine kontaktlose Bedienung bzw. Ansteuerung von Funktionen eines medizintechnischen Geräts ermöglicht wird. Außerdem erlaubt eine Nutzung einer am Körper mitführbaren Befehlsübermittlungseinrichtung eine Ansteuerung unterschiedlicher medizintechnischer Geräte auf dieselbe Art und Weise. Herkömmlich weisen Bedienschalter mechanischer Geräte unterschiedlichen Typs unterschiedliche Ausprägungen ihrer Lage, ihres Druckpunkts, des nötigen Kraftaufwands für eine Betätigung und der durch die Betätigung realisierten Funktionen auf. Mit der erfindungsgemäßen Verschiebung der Eingabeschnittstelle zu dem Benutzer kann nun eine intuitivere Bedienung mit einem vereinheitlichten und geräteübergreifenden Bedienkonzept umgesetzt werden. Aufgrund der Verlagerung der Eingabeschnittstelle zu dem Körper der Bedienperson hin kann auch die Aufmerksamkeit des Nutzers während einer Bedienung eines medizintechnischen Geräts bei der aktuellen parallelen Tätigkeit verbleiben. Beispielsweise muss für eine Betätigung des medizintechnischen Geräts nicht die Aufmerksamkeit von dem Patienten abgezogen werden. Bei sicherheitsrelevanten Behandlungsschritten, bei denen volle Aufmerksamkeit benötigt wird, wird also vorteilhaft kein zusätzliches Personal benötigt, welches parallel dazu die Bedienung des medizintechnischen Geräts übernimmt.

Beispielsweise können bei unterschiedlichen Geräten analoge Funktionen auf dieselbe Weise bzw. mit derselben Geste ausgelöst werden, ohne dass ein Benutzer die entsprechenden unterschiedlich ausgebildeten Bedienelemente an den einzelnen unterschiedlich strukturierten Geräten finden muss. Auf diese Weise wird die Einarbeitungszeit reduziert. Beispielsweise fällt das Einarbeiten in unterschiedliche Benutzeroberflächen unterschiedlicher medizintechnischer Geräte weg, da die Steuerung analoger Funktionen stets gleichartig durch dieselben Körperbewegungen erfolgen kann. Vorteilhaft wird durch die erfindungsgemäße Befehlsübermittlungseinrichtung die Bedienung eines Medizintechnikgeräts intuitiv in einen Arbeitsablauf integriert. Außerdem ist eine Mehrfach- und Wiederverwendbarkeit auch für unterschiedliche Anwendungen möglich, wenn die Auswertungseinheit programmierbar ausgestaltet wird.

Das erfindungsgemäße Befehlsübermittlungssystem weist eine erste erfindungsgemäße Befehlsübermittlungseinrichtung mit Gestenerkennung auf. Die erste erfindungsgemäße Befehlsübermittlungseinrichtung ist an der Bedienperson fixiert und ist als Master-Einrichtung für den bidirektionalen Datenaustausch mit mindestens einer Slave-Einrichtung ausgebildet. D.h., die erste erfindungsgemäße Befehlsübermittlungseinrichtung funktioniert nach dem Master-Slave-Prinzip als Master. Es können also Befehle nur übertragen werden, wenn die Befehlsübertragung über die erste erfindungsgemäße Befehlsübermittlungseinrichtung freigeschaltet wurde.

Weiterhin ist die Gestensensoreinheit der ersten Befehlsübermittlungseinrichtung aktiviert, d.h. die erste Befehlsübermittlungseinrichtung reagiert auf Gestensteuerung und ist dazu eingerichtet, einen ermittelten Befehl, vorzugsweise einen Initialisierungsbefehl an eine zweite, vorzugsweise erfindungsgemäße Befehlsübermittlungseinrichtung zu übermitteln.

Die zweite, vorzugsweise erfindungsgemäße Befehlsübermittlungseinrichtung ist ebenfalls Teil des erfindungsgemäßen Befehlsübermittlungssystems und ist entfernt von der Bedienperson, vorzugsweise an einer zu bedienenden medizintechnischen Einrichtung angeordnet. Die zweite Befehlsübermittlungseinrichtung ist als Slave-Einrichtung für den bidirektionalen Datenaustausch mit einer Master-Einrichtung ausgebildet und sie umfasst eine Auswertungseinheit, die mindestens eine Untereinheit zur Erkennung von Befehlsinhalten auf Basis von Sensordaten umfasst. Bevorzugt umfasst die Auswertungseinheit eine Gestenerkennungseinheit, die zur Erfassung einer Geste der Bedienperson aus einer Distanz zu der Bedienperson aktiviert ist. Die Auswertungseinheit umfasst auch bevorzugt eine Spracherkennungseinheit mit einer Spracherkennungsfähigkeit zur Erkennung von Sprachinhalten auf Basis von akustischen Sensordaten.

Die Auswertungseinheit weist also die Fähigkeiten zur Erfassung einer Geste oder einer akustischen Information der Bedienperson aus einer Distanz zu der Bedienperson auf. Diese Funktionen sind von der als Master-Einrichtung ausgebildeten ersten Befehlsübermittlungseinrichtung aus aktivierbar. Die zweite Befehlsübermittlungseinrichtung umfasst also bevorzugt auch eine Spracherkennungsfähigkeit, mit der ein Sprachbefehl von der Bedienperson ausgewertet werden kann. Weiterhin ist die zweite Befehlsübermittlungseinrichtung dazu eingerichtet, einen von der ersten Befehlsübermittlungseinrichtung empfangenen Befehl, beispielsweise einen Sprachbefehl, zu empfangen und auf Basis dieses Befehls einen Steuerbefehl an die medizintechnische Einrichtung zu übermitteln bzw. diese auf Basis des empfangenen Befehls anzusteuern. Vorteilhaft lässt sich die zweite Befehlsübermittlungseinrichtung zunächst durch einen Befehl, der über die erste erfindungsgemäße Befehlsübermittlungseinrichtung, beispielsweise durch eine Bewegung bzw. Geste der Bedienperson initiiert, gegeben wurde, dazu initialisieren, empfangsbereit zu sein. Anschließend steuert die Bedienperson die medizintechnische Einrichtung durch einen zusätzlichen Steuerbefehl, der entweder direkt, beispielsweise durch einen Sprachbefehl oder eine Geste erzeugt wird, entweder direkt durch Erfassen des Sprachbefehls oder der Geste durch die zweite Befehlsübermittlungseinrichtung oder durch ein Erfassen dieses zusätzlichen Befehls durch die erste Befehlsübermittlungseinrichtung und ein anschließendes Übertragen des erfassten zusätzlichen Befehls an die zweite Befehlsübermittlungseinrichtung, die dann die medizintechnische Einrichtung gemäß dem empfangenen oder erfassten Befehl ansteuert.

Vorteilhaft lässt sich durch die beschriebene zweistufige Vorgehensweise eine erhöhte Zuverlässigkeit bei der Bedienung der medizintechnischen Einrichtung erreichen, da diese erst dann gesteuert werden kann, wenn vorab ein Initialisierungsbefehl von der Bedienperson gegeben wurde.

Das erfindungsgemäße Operationssystem umfasst eine bildgebende medizintechnische Einrichtung zur Überwachung einer Operation eines Patienten. Insbesondere kann mit einer bildgebenden medizintechnischen Einrichtung ein im Inneren eines Patienten liegender Operationsbereich vor oder während einer Operation dargestellt werden. Das erfindungsgemäße Operationssystem umfasst auch eine Operationseinrichtung. Eine solche Operationseinrichtung kann zum Beispiel eine Vorrichtung zur minimalinvasiven Operation, Gerätschaften zur Behandlung und Überwachung eines Patienten, wie zum Beispiel eine Überwachung der wichtigsten Körperfunktionen, sowie einen Operationstisch umfassen. Das erfindungsgemäße Operationssystem umfasst auch eine erfindungsgemäße Befehlsübermittlungseinrichtung oder ein erfindungsgemäßes Befehlsübermittlungssystem, das dazu eingerichtet ist, einen Steuerbefehl an die bildgebende medizintechnische Einrichtung zu übermitteln und/oder die bildgebende medizintechnische Einrichtung mit diesem Befehl anzusteuern.

Vorteilhaft lässt sich mit dem erfindungsgemäßen Operationssystem parallel neben der Durchführung einer Operation durch ein und dieselbe Person auch eine medizinische Bildgebung durchführen, wobei der Chirurg zum Beispiel seine Hände benutzt, um die Operation auszuführen, mit seinen Augen auf dem Bildschirm der medizintechnischen bildgebenden Einrichtung den Operationsvorgang im Inneren des Patienten verfolgt und überwacht und gleichzeitig mit einem anderen Körperteil die Bildgebung steuert. Mithin muss der Chirurg zur Ansteuerung der medizintechnischen bildgebenden Einrichtung seinen Blick nicht von dem Bildschirm abwenden und muss auch nicht den Operationsvorgang unterbrechen. Beispielsweise kann der Chirurg die Bilddarstellung, den Bildausschnitt und die Lokalisierung des abzubildenden Bereichs während des Operationsvorgangs dynamisch anpassen, um eine optimale Darstellung für den jeweiligen Teilabschnitt einer Operation zu erhalten.

Bei dem erfindungsgemäßen Befehlsübermittlungsverfahren wird eine Bewegung einer Bedienperson durch eine an der Bedienperson angeordnete Gestenerkennungseinheit erfasst. Auf Basis der erfassten Bewegung wird ein Befehl der Bedienperson identifiziert und der identifizierte Befehl wird an eine zu bedienende medizintechnische Einrichtung übermittelt.

Das erfindungsgemäße Befehlsübermittlungsverfahren teilt die Vorteile der erfindungsgemäßen Befehlsübermittlungseinrichtung.

Die wesentlichen Komponenten der erfindungsgemäßen Befehlsübermittlungseinrichtung können zum überwiegenden Teil in Form von Softwarekomponenten ausgebildet sein. Dies betrifft insbesondere die Auswertungseinheit und Teile der Befehlsübermittlungseinheit.

Grundsätzlich können diese Komponenten aber auch zum Teil, insbesondere wenn es um besonders schnelle Berechnungen geht, in Form von softwareunterstützter Hardware, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden.

Eine weitgehend softwaremäßige Realisierung hat den Vorteil, dass auch schon bisher verwendete Datenverarbeitungseinrichtungen unter Nachrüstung benötigter Hardware, wie zum Beispiel eine Sensoreinheit und eine Fixierungseinheit, auf einfache Weise durch ein Software-Update dazu eingerichtet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung einer Datenverarbeitungseinrichtung ladbar ist und Programmabschnitte umfasst, um alle Schritte des erfindungsgemäßen Verfahrens auszuführen, wenn das Computerprogramm in der Datenverarbeitungseinrichtung ausgeführt wird.

Ein solches Computerprogrammprodukt kann neben dem Computerprogramm gegebenenfalls zusätzliche Bestandteile wie z.B. eine Dokumentation und/oder zusätzliche Komponenten, auch Hardware-Komponenten, wie z.B. Hardware-Schlüssel (Dongles etc.) zur Nutzung der Software, umfassen.

Zum Transport zur Speichereinrichtung der Datenverarbeitungseinrichtung und/oder zur Speicherung an der Datenverarbeitungseinrichtung kann ein computerlesbares Medium, beispielsweise ein Memorystick, eine Festplatte oder ein sonstiger transportabler oder fest eingebauter Datenträger dienen, auf welchem die von einer Datenverarbeitungseinrichtung, beispielsweise eine Rechnereinheit, einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Die abhängigen Ansprüche sowie die nachfolgende Beschreibung enthalten jeweils besonders vorteilhafte Ausgestaltungen und Weiterbildungen der Erfindung. Dabei können insbesondere die Ansprüche einer Anspruchskategorie auch analog zu den abhängigen Ansprüchen einer anderen Anspruchskategorie weitergebildet sein. Zudem können im Rahmen der Erfindung die verschiedenen Merkmale unterschiedlicher Ausführungsbeispiele und Ansprüche auch zu neuen Ausführungsbeispielen kombiniert werden.

Bevorzugt umfasst die Sensoreinheit der erfindungsgemäßen Befehlsübermittlungseinrichtung eine interne Gestensensoreinheit für eine Gestenerkennung. Als interne Gestensensoreinheit soll eine Gestensensoreinheit verstanden werden, die innerhalb eines Kleidungsstücks einer Bedienperson oder eines von der Bedienperson getragenen Objekts angeordnet ist, also in dieses Objekt integriert ist. Eine Gestenerkennung bezieht sich im Allgemeinen auf eine gezielte Bewegung einer Person, mit der diese Person eine Information, vorzugsweise einen Steuerbefehl übermitteln möchte. Eine solche Geste kann zum Beispiel eine Bewegung einer Person mit einer oder beiden Händen, mit einem Fuß oder beiden Füßen, mit einem Bein oder beiden Beinen, mit einem Auge oder beiden Augen oder eine Kopfbewegung umfassen. Eine Geste kann auch eine vorab festgelegte Kombination und/oder Abfolge aus einer oder mehreren der genannten Bewegungen umfassen. Vorteilhaft ist die Sensoreinheit von außen nicht sichtbar bzw. nach außen hin geschützt, so dass die Bedienperson nicht in ihren Bewegungsabläufen beeinträchtigt ist und die Gestensensoreinheit gut gegen mechanische Beschädigung geschützt ist. Die interne Gestensensoreinheit kann zum Beispiel in einem Kleidungsstück angeordnet sein. Vorteilhaft kann eine Geste einer die Befehlsübermittlungseinrichtung bei sich tragenden Person automatisiert erfasst und zur Ansteuerung einer medizintechnischen Einrichtung genutzt werden.

Die Gestenerkennung kann bevorzugt zur Erfassung und Erkennung einer "absichtlichen" Bewegung genützt werden. Wird eine solche "absichtliche" Bewegung anhand des Bewegungsmusters identifiziert, so kann zum Beispiel ein Befehl zur Vorbereitung einer Aktion gegeben werden. Beispielsweise kann eine Spracheingabe zum Geben differenzierterer Befehle zur Steuerung einer medizintechnischen Einrichtung freigeschaltet werden.

Zur weiteren Erhöhung der Zuverlässigkeit im Sinne einer verbesserten Detektionswahrscheinlichkeit einer Bedienung einer medizintechnischen Einrichtung kann ein vorbestimmtes Bewegungsmuster ein Bewegungsmuster einer sogenannten "sicheren absichtlichen" Bewegung aufweisen. Um die Erkennung eines Befehls besonders sicher zu machen, kann eine solche "sichere absichtliche" Bewegung eine komplexere Bewegungsabfolge umfassen, die nicht leicht mit einer zufälligen natürlichen Bewegung verwechselt werden kann. Beispielswiese kann ein entsprechendes Bewegungsmuster ein Tippen eines Fußes mit einer vorbestimmten Abfolge, wie zum Beispiel Spitze, Ferse, Spitze, oder ein mehrmaliges Tippen mit der Ferse umfassen.

Besonders bevorzugt, um eine sehr sichere Befehlsübermittlung zu erreichen, ist es, mehrere Bewegungssensoren an verschiedenen Körperstellen zu verteilen. Beispielsweise kann in beiden Schuhen einer Person jeweils mindestens ein Sensor oder gar eine vollständige Befehlsübermittlungseinrichtung integriert sein. Mit einer solchen Anordnung könnten komplexe Bewegungssequenzmuster zweier Füße erzeugt und erkannt werden. Noch komplexere Bewegungsmuster wären zum Beispiel durch eine Anordnung von Bewegungssensoren an mehreren einzelnen Zehen möglich. Durch eine solche Erhöhung der Komplexität der Ansteuerung ist zum einen eine verbesserte Sicherheit bei der Identifizierung von Befehlen anhand von Körperbewegungen möglich und zum anderen lassen sich auch differenzierte und komplexere Befehle oder Befehlssequenzen übermitteln, wodurch eine detaillierte Fernsteuerung eines medizintechnischen Geräts ermöglicht wird.

Die Gestenerkennung kann bevorzugt durch Erfassung einer Bewegung der Sensoreinheit oder einer mechanischen Einwirkung auf die Sensoreinheit erfolgen.

Binäre Aktionen, wie zum Beispiel das Einschalten oder Ausschalten können durch eine Piezokeramik einer Sensoreinheit als Ersatz für einen mechanischen Schalter erfasst bzw. ausgelöst werden.

Größere und beliebige Variationen von auszuübenden Aktionen, wie zum Beispiel ein Verfahren nach oben oder nach unten oder nach links oder nach rechts können zum Beispiel mit einer Beschleunigungsmesseinrichtung realisiert werden.

Weiterhin kann als Sensoreinheit auch eine Einrichtung zum Messen einer plastischen Verformung oder einer Änderung eines Magnetfelds eingesetzt werden.

Bevorzugt ist die Fixierungseinheit der erfindungsgemäßen Befehlsübermittlungseinrichtung dazu ausgebildet, die Gestensensoreinheit an oder in einem Kleidungsstück, vorzugsweise in einem Schuh, zu fixieren. Die Fixierungseinheit kann also in das Kleidungsstück integrierbar sein, bevorzugt in einer Sohle eines Schuhs, besonders bevorzugt als Einlegesohle in einem Schuh, integrierbar sein. Bevorzugt ist die Fixierungseinheit der erfindungsgemäßen Befehlsübermittlungseinrichtung als Aussparung in der Sohle des Schuhs ausgebildet. Besonders bevorzugt ist die Fixierungseinheit, abnehmbar oder austauschbar, beispielsweise als textiler Überzieher ausgebildet. Vorteilhaft kann die Befehlsübermittlungseinrichtung quasi passiv von der Bedienperson getragen werden, ohne dass diese sich aktiv darum kümmern muss, beispielsweise die Befehlsübermittlungseinrichtung festhalten muss. Mithin hat die Bedienperson ihre Extremitäten, insbesondere ihre Hände, für parallel zu erfüllende Aufgaben zur Verfügung. Bei einer Integration der Befehlsübermittlungseinrichtung in einen Schuh oder einer Befestigung der Befehlsübermittlungseinrichtung an einem Schuh ergeben sich erweiterte Möglichkeiten und ein besserer Komfort gegenüber der Steuerung mit den Händen, da die Hände für parallele Tätigkeiten frei sind. Ein Gesten-schuhsensor ermöglicht also zusätzliche Freiheitsgrade bzw. Gestaltungsmöglichkeiten für einen Workflow einer medizinischen Untersuchung. Insbesondere können mehre Aktionen parallel gesteuert bzw. ausgeführt werden. Ein Beispiel für eine Ansteuerung im Rahmen eines solchen Workflows ist eine Ansteuerung einer Bilddarstellung bei einem medizintechnischen Bildgebungsverfahren. Dabei kann zum Beispiel eine Betrachtungsperspektive oder ein Zoomeffekt mit Hilfe einer Geste gesteuert werden und parallel kann mit den Händen eine Untersuchung oder Behandlung eines Patienten vorbereitet oder durchgeführt werden.

Auch bevorzugt umfasst die Befehlsübermittlungseinheit der erfindungsgemäßen Befehlsübermittlungseinrichtung eine Funkübertragungseinheit zum Übermitteln des identifizierten Befehls an die zu bedienende medizintechnische Einrichtung.

Vorteilhaft kann die Befehlsübermittlung kabellos erfolgen, so dass kein störendes Übertragungskabel benötigt wird, was zum Beispiel eine Störquelle oder eine potentielle Unfallursache für eine Person darstellen kann.

Die erfindungsgemäße Befehlsübermittlungseinrichtung weist bevorzugt eine Energieerzeugungs- und speichereinheit auf, welche dazu eingerichtet ist, aus der Bewegungsenergie der Bedienperson elektrische Energie zu erzeugen. Ein solcher Vorgang wird auch als "Energy Harvesting" (zu Deutsch Energieernten) bezeichnet und betrifft die Gewinnung kleiner Mengen von elektrischer Energie aus Quellen wie der Umgebungswärme, Vibrationen oder Luftströmungen für mobile Geräte mit geringer elektrischer Leistung. Die für die Erzeugung dieser Energie eingesetzten Strukturen werden auch als Nanogenerator bezeichnet. Energieernten vermeidet bei Drahtlostechnologien Einschränkungen durch kabelgebundene Stromversorgung oder Batterien. Die Energieerzeugungs- und Speichereinheit ist bevorzugt als interne elektronische Energieerzeugungs- und speichereinheit ausgebildet, welche vorzugsweise eine Piezokeramik aufweist, die die Energie der Bewegung, beispielsweise der Laufbewegung des tragenden Nutzers in elektrische Energie umsetzt und speichert.

Besonders bevorzugt ist die erfindungsgemäße Befehlsübermittlungseinrichtung für den Fall, dass sie als Slave-Einheit ausgeführt ist, derart ausgebildet, dass die Sensoreinheit eine akustische Sensoreinheit umfasst und die Auswertungseinheit eine Spracherkennungseinheit zum Erkennen und Interpretieren von Sprachbefehlen der Bedienperson umfasst. Mit einer Spracherkennung wird gesprochene Sprache der automatischen Datenerfassung zugänglich gemacht, es kann also eine Dateneingabe per Sprache erfolgen, um eine Ansteuerung einer medizintechnischen Einrichtung auszuführen. Vorteilhaft kann ein Sprachbefehl komplexere Inhalte übermitteln als eine Geste. Außerdem müssen keine zeichenhaften Gesten auswendig gelernt werden, um eine medizintechnische Einrichtung anzusteuern.

Besonders vorteilhaft hat eine Bedienperson ihre Extremitäten, insbesondere die Hände, frei für parallel auszuführende Tätigkeiten.

Auch bevorzugt ist die Befehlsübermittlungseinrichtung dazu eingerichtet, dass die interne Gestenerkennung eine der folgenden Funktionsbefehle erkennt:
- Scrollen von Bildern auf einer Bildanzeige eines medizintechnischen Geräts bzw. das Durchlaufen einer Sequenz solcher Bilder,
- die Veränderung des Bildkontrasts,
- das Hineinzoomen in oder Herauszoomen aus einer Bilddarstellung,
- Ändern von spezifischen Parametern eines medizintechnischen Geräts.

Vorteilhaft kann parallel zu einer anderen Tätigkeit mit Hilfe einer Geste eine Anpassung einer Bildanzeige oder eines anderen Parameters eines medizintechnischen Geräts vorgenommen werden, ohne dass die Bedienperson dadurch von ihrer primären Tätigkeit abgelenkt wird.

Bevorzugt ist die erfindungsgemäße Befehlsübermittlungseinrichtung dazu eingerichtet, der Bedienperson in Antwort auf einen erkannten Befehl eine haptische Rückmeldung zu übermitteln. Vorteilhaft erhält die Bedienperson eine Information darüber, dass ihr Befehl erkannt wurde und übermittelt wurde. Für eine solche haptische Rückmeldung umfasst die erfindungsgemäße Befehlsübermittlungseinrichtung bevorzugt einen Aktor, der eine mechanische Bewegung ausführen kann, mit der einer Bedienperson signalisiert wird, dass ein durch eine Bewegung, vorzugsweise eine Geste, gegebener Befehl von der Befehlsübermittlungseinrichtung erkannt und weitergegeben wurde. Eine solche Bewegung des Aktors kann zum Beispiel eine Vibration umfassen, welche auf den Körper einer Bedienperson übertragen wird und von dieser haptisch wahrgenommen wird. Vorteilhaft wird eine Fernsteuerung durch eine Bewegung oder Geste, welche herkömmlich nicht-haptisch ausgestaltet ist, da ja mit der Bewegung bzw. Geste kein Bedienschalter berührt wird und daher keine durch die Schaltermechanik verursachte mechanische Rückmeldung infolge eines Druckpunkts oder einer Gegenkraft gegen einen Bedienakt eintritt, um eine haptische Rückmeldung ergänzt, so dass eine Person, welche eine Steuerungsbewegung, vorzugsweise eine Geste ausführt, einen ähnlichen haptischen Eindruck erhält wie bei einer direkten Betätigung eines Schalters oder einer Taste. Da die Befehlsübermittlungseinrichtung ja direkt an dem die Steuerbewegung ausführenden Körperteil befestigt ist, wird die Rückmeldung an der Stelle des Körpers einer Bedienperson gegeben, von der die Steuerbewegung ausgeführt wurde, was dem Erleben eines herkömmlichen Steuervorgangs durch eine direkte mechanische Betätigung von Hand oder durch eine Betätigung mit einem anderen Körperteil entspricht.

Mithin muss sich eine Bedienperson bei einer Fernbedienung einer medizintechnischen Einrichtung hinsichtlich der Haptik des Vorgangs nicht umgewöhnen. Außerdem muss die Bedienperson aufgrund der vorzugsweise sofortigen haptischen Rückmeldung nicht auf eine Ausführung seiner Aktion warten, um festzustellen, ob sein Steuerungsbefehl erkannt wurde, und gegebenenfalls erst dann eine Steuerungsbewegung wiederholen. Mithin spart die Bedienperson durch die direkte Rückmeldung Zeit und kann für den Fall, dass es keine sofortige Rückmeldung gibt, sicher sein, dass die Steuerungsbewegung nicht erkannt wurde, und die Steuerungsbewegung sofort wiederholen oder andernfalls, falls sie eine sofortige Rückmeldung erhält, mit einer anderen Steuerungsbewegung ohne Zeitverzögerung fortfahren.

Bevorzugt ist die zweite Befehlsübermittlungseinrichtung des erfindungsgemäßen Befehlsübermittlungssystems dazu eingerichtet, nach Aktivierung der Spracherkennungseinheit einen Sprachbefehl von der Bedienperson zu detektieren und zu identifizieren und auf Basis des identifizierten Sprachbefehls einen Steuerbefehl an die medizintechnische Einrichtung zu übermitteln. Die Aktivierung kann vorzugsweise durch eine von der ersten Befehlsübermittlungseinrichtung übermittelten Befehl, beispielsweise auf Basis einer Geste der Bedienperson, erfolgen. Dadurch wird eine gewisse Schutzfunktion erreicht. D.h. es wird sichergestellt, dass ein Sprachbefehl auch zur Ansteuerung der medizintechnischen Einrichtung gegeben wird.

Beispielsweise kann die Gestenerkennungseinheit oder die Spracherkennungseinheit eine Fähigkeit zur Erkennung von Bestätigungssignalen folgender Funktionen umfassen:
- eine Strahlungsauslösung,
- eine Magnetfeldauslösung
- Bewegungen des medizintechnischen Geräts.

Die Exposition durch Röntgenstrahlen ist formal juristisch gesehen Körperverletzung und darf daher nur im Einverständnis mit dem Patienten eingesetzt werden.

Die Auslösung von Gradienten für eine MRT-Aufnahme kann zu einer Beeinträchtigung des Körpers akustischer Natur verführen, da eine Aufnahme ohne Gehörschutz als sehr laut empfunden wird.

Schäden durch motorisierte medizinische Geräte von beispielsweise Patiententisch mobilem C-Bogen sind durch Sicherheitstechnik auszuschließen.

Mithin wird der Patient durch eine zweistufige Ansteuerung mit einer Initialisierung bzw. Aktivierung einer Befehlsübermittlung vor unbeabsichtigten Schäden durch eine erhöhte Strahlenexposition, eine Magnetfeldexposition oder eine mechanische Einwirkung durch eine Bewegung eines medizintechnischen Geräts vorteilhaft geschützt.

Ebenfalls bevorzugt ist die Gestenerkennungseinheit der ersten Befehlsübermittlungseinrichtung des erfindungsgemäßen Befehlsübermittlungssystems dazu ausgebildet, eine der folgenden Gesten zu erfassen:
- Hände oder Finger greifen, loslassen, zoomen, bewegen
- Augen bewegen sich nach links, rechts, oben, unten, Blinzeln,
- Kopfbewegung nach links oder rechts.

Vorteilhaft lassen sich der Befehlsraum und die Eingabemöglichkeiten mit den beschriebenen Gesten erweitern. Manche Gesten mit anderen Körperteilen sind für bestimmte Workflows besonders gut geeignet. Als klaren Vorteil aller hier aufgeführter Eingabemöglichkeiten ist anzusehen, dass außer dem Tragen einer speziellen Brille, ausgestattet mit Kamera(s) und Beschleunigungssensor, kein spezielles Gerät getragen bzw. an der Person angebracht werden muss, beispielsweise ein Sensorhandschuh oder dergleichen. Die Bewegungsfreiheit und bisherigen Gewohnheiten des Nutzers werden damit nicht eingeschränkt, da sich die Sensorik zur Erfassung von Aktionen an den Nutzer anpasst und nicht umgekehrt.

Bevorzugt ist die erste Befehlsübermittlungseinrichtung des erfindungsgemäßen Befehlsübermittlungssystems dazu eingerichtet, der Bedienperson in Antwort auf einen erkannten Befehl eine haptische Rückmeldung zu übermitteln. Vorteilhaft erhält die Bedienperson eine Information darüber, dass ihr Befehl erkannt wurde und übermittelt wurde.

Im Folgenden wird die Erfindung anhand der in den Figuren dargestellten Ausführungsbeispiele näher beschrieben und erläutert.

Es zeigen:
FIG 1 eine schematische Darstellung eines Schuhs mit zwei Befehlsübermittlungseinrichtungen gemäß einem Ausführungsbeispiel der Erfindung,
FIG 2 eine schematische Darstellung einer Befehlsübermittlungseinrichtung gemäß einem Ausführungsbeispiel der Erfindung,
FIG 3 eine schematische Darstellung einer zweiten Befehlsübermittlungseinrichtung eines Befehlsübermittlungssystems gemäß einem Ausführungsbeispiel der Erfindung, welche als festinstallierte Empfangseinrichtung und Aktoreinrichtung ausgebildet ist,
FIG 4 eine schematische Darstellung eines Gesten-Schuhsensors gemäß einem Ausführungsbeispiel der Erfindung,
FIG 5 eine schematische Darstellung eines Computertomographiesystems mit einem Befehlsübermittlungssystem gemäß einem Ausführungsbeispiel der Erfindung,
FIG 6 ein Flussdiagramm, welches ein Befehlsübermittlungsverfahren gemäß einem Ausführungsbeispiel der Erfindung veranschaulicht
FIG 7 eine schematische Darstellung eines Befehlsübermittlungssystems gemäß einem Ausführungsbeispiel der Erfindung,
FIG 8 eine schematische Darstellung eines Befehlsübermittlungssystems gemäß einem Ausführungsbeispiel.

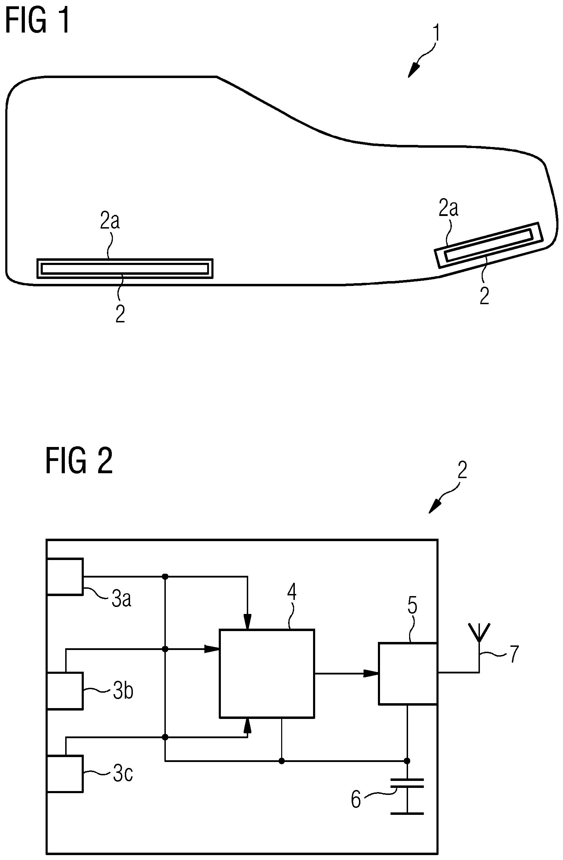

In FIG 1 ist ein Schuh 1 mit zwei Befehlsübermittlungseinrichtungen 2 gemäß einem Ausführungsbeispiel der Erfindung gezeigt. Die Befehlsübermittlungseinrichtungen 2 sind als drahtlose Schuh-Sensoren ausgebildet und sind jeweils durch Fixierungseinheiten 2a, welche die Befehlsübermittlungseinrichtungen 2 jeweils umgeben, in eine Schuhsohle integriert. Eine Bewegung des Schuhs 1 wird von einer Befehlsübermittlungseinrichtung 2 durch Sensoren (siehe FIG 2) erfasst und ausgewertet. Auf Basis der Auswertung erfolgt eine Erzeugung eines Befehls, der an eine medizintechnische Einrichtung übermittelt wird, so dass eine Steuerung, insbesondere ein Ein- oder Ausschalten einer medizintechnischen Einrichtung ferngesteuert erfolgt, ohne dass die Bedienperson sich der medizintechnischen Einrichtung nähern muss oder die Hände für einen Schaltvorgang benutzen muss.

In FIG 2 ist eine Befehlsübermittlungseinrichtung 2 gemäß einem Ausführungsbeispiel der Erfindung schematisch dargestellt. Die Befehlsübermittlungseinrichtung 2 umfasst eine Mehrzahl von Sensoren 3a, 3b, 3c, welche eine Bewegung oder eine andere physikalische Information erfassen. Die Sensoren 3a, 3b, 3c können zum Beispiel jeweils einen Drucksensor umfassen, welcher eine elektrische Spannungsänderung in Antwort auf eine externe mechanische Krafteinwirkung misst. Die erzeugten Sensorsignale werden an eine Auswertungseinheit 4, beispielsweise ein Mikrokontroller 4, mit ultraniedrigem Energieverbrauch übermittelt, die ebenfalls Teil der Befehlsübermittlungseinrichtung 2 ist. Der Mikrokontroller 4 erzeugt auf Basis der Sensorsignale ein Befehlssignal. Die Befehlsübermittlungseinrichtung 2 umfasst auch eine Sende-Empfangseinheit, im einfachsten Fall ein RF-Sender 5, und eine Antenne 7 zum Übertragen eines auf Basis einer von der Auswertungseinheit 4 ermittelten Willensäußerung einer Bedienperson erzeugten Befehlssignals.

Die Sende-Empfangseinheit 5 kann auch als Schnittstelle zum Empfang zusätzlicher externer Sensorsignale, die von externen Einheiten an die Befehlsübermittlungseinrichtung 2 übermittelt werden, eingesetzt werden. Beispielsweise kann eine externe akustische Sensoreinheit sowie eine externe Bilddatenerfassungseinheit Informationen über Lautäußerungen und Bewegungen oder Haltungsänderungen einer Bedienperson erfassen und an die Befehlsübermittlungseinrichtung 2 übermitteln.

Die akustischen Daten sowie die Bilddaten können dann von der Auswertungseinheit 4 zusätzlich zu den von den internen Sensoren 3a, 3b, 3c erfassten Sensordaten ausgewertet werden, um eine Willensäußerung einer Bedienperson zu ermitteln.

Die Befehlsübermittlungseinrichtung 2 umfasst überdies eine interne elektronische Energieerzeugungs- und Energiespeichereinheit 6. Die Energieerzeugungs- und Energiespeichereinheit 6 umfasst ein piezokeramisches Element, mit dem durch eine mechanische Krafteinwirkung auf das genannte Element elektrische Energie erzeugt wird und speicherbar ist. Beispielsweise kann eine Bewegung eines Schuhs, eines Beins oder eines Arms dazu genutzt werden, im Rahmen eines sogenannten Energy-Harvesting-Prozesses elektrische Energie zu erzeugen. Diese wird dann in einem elektrischen Energiespeicher, beispielsweise einem Kondensator oder einem Akkumulator gespeichert und dazu genutzt, den Mikrokontroller 4 bzw. die Auswertungseinheit 4, den Sender 5 und die Sensoren 3a, 3b, 3c mit elektrischer Energie zu versorgen.

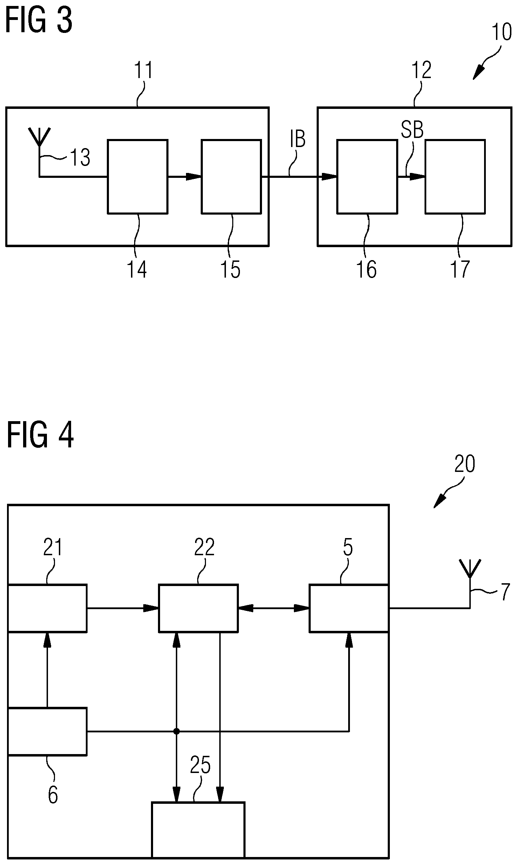

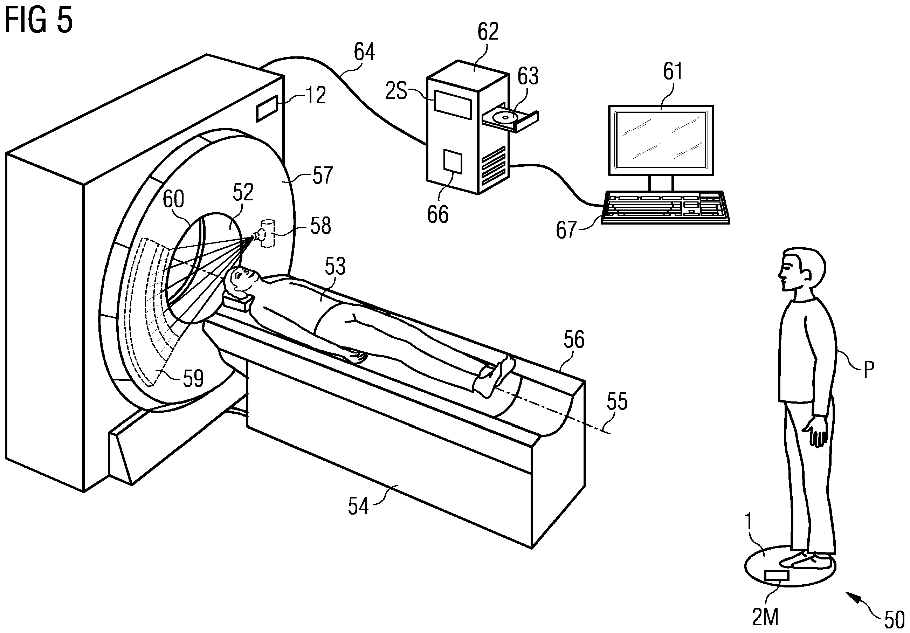

In FIG 3 ist eine festinstallierte Empfangseinrichtung 10 eines Befehlsübermittlungssystems gemäß einem Ausführungsbeispiel der Erfindung veranschaulicht. Ein solches Befehlsübermittlungssystem 50 (siehe FIG 5) umfasst die in FIG 2 gezeigte Befehlsübermittlungseinrichtung 2 sowie die in FIG 3 gezeigte fest installierte Empfangseinrichtung 10. Die fest installierte Empfangseinrichtung 10 ist zum Beispiel an einer medizintechnischen Einrichtung, wie zum Beispiel das in FIG 5 gezeigte CT-System 50, angeordnet. Die fest installierte Empfangseinrichtung 10 umfasst eine drahtlose Empfangseinheit 11 und ein Aktorsystem 12. Die drahtlose Empfangseinheit 11 umfasst eine Antenne 13 und einen Empfänger 14, zum Beispiel einen RF-Empfänger, zum Empfangen eines Befehlssignals von einer an einem Schuh 1 installierten Befehlsübermittlungseinrichtung 2. Weiterhin umfasst die drahtlose Empfangseinheit 11 auch einen Mikrokontroller 15, welcher dazu eingerichtet ist, ein von der drahtlosen Empfangseinheit 11 empfangenes Befehlssignal auszuwerten. Auf Basis des Befehlssignals wird an das Aktorsystem 12 ein Initialisierungsbefehl IB gegeben. Das Aktorsystem 12 umfasst einen System-Mikrokontroller 16 sowie einen Aktor 17. Der System-Mikrokontroller 16 empfängt den Initialisierungsbefehl IB und übermittelt einen Steuerbefehl an einen Aktor bzw. einen Schalter, der betätigt wird, um einen Betrieb einer medizintechnischen bildgebenden Einrichtung zu starten.

In FIG 4 ist eine schematische Darstellung eines Gesten-Schuhsensors 20 gemäß einem Ausführungsbeispiel der Erfindung veranschaulicht. Der Gesten-Schuhsensor 20 umfasst eine Inertialsensorik 21, mit der eine Beschleunigung, eine Drehung oder ein Magnetfeld gemessen werden können. Eine solche Sensorik kann zum Beispiel Bauteile umfassen, in denen sogenannte MEMS in ICs integriert sind und die eine serielle Schnittstelle zu einem Microcontroller bieten. Die erfassten Sensorsignale werden von der Inertialsensorik 21 an einen Mikrokontroller 22, der besonders wenig Energie verbraucht, übermittelt. Der Mikrokontroller 22 erzeugt auf Basis des von der Inertialsensorik 21 erzeugten Sensorsignals ein Gesten-Befehlssignal und übermittelt das Gesten-Befehlssignal an eine Sende-Empfangs-Einheit 5, die ebenfalls Teil des Gesten-Such-Sensors 20 ist.

Die Sende-Empfangs-Einheit 5 erzeugt ein Initialisierungsbefehlssignal, welches mit Hilfe einer Antenne 7 an eine festinstallierte Empfangseinrichtung übermittelt wird. Teil des Gesten-Schuhsensors 20 ist auch eine Energieversorgung 6, die beispielsweise in der in FIG 2 gezeigten Art und Weise ausgebildet sein kann. Darüber hinaus umfasst der Gesten-SchuhSensor 20 auch eine Einheit 25 zur Übermittlung eines haptischen Feedbacks an eine Bedienperson. Gibt die Bedienperson einen Initialisierungsbefehl mit Hilfe einer Geste, so erfasst die Inertialsensorik 21 die Bewegung und übermittelt das erfasste Sensorsignal an den Mikrokontroller 22. Der Mikrokontroller 22 wertet das Sensorsignal aus und für den Fall, dass er eine Geste erkennt, die einen Initialisierungsbefehl kennzeichnet, gibt er ein entsprechendes Befehlssignal an die Sende-Empfangseinheit 5 aus und übermittelt zusätzlich ein Steuersignal an die Einheit 25 zur Übermittlung eines haptischen Feedbacks. Die Einheit 25 zur Übermittlung eines haptischen Feedbacks erzeugt daraufhin ein mechanisches Signal, beispielsweise eine Vibrationsbewegung, mit der die Bedienperson bzw. der Träger des Gesten-Schuh-Sensors 20 darüber informiert wird, dass der von ihm durch eine Geste erzeugte Befehl erkannt und weitergegeben wurde. Mit dem Befehl wird dann zum Beispiel eine Eingabe für eine medizintechnische Bildgebungseinrichtung freigeschaltet. Anschließend kann die Bedienperson durch eine weitere Geste, beispielsweise durch eine Handbewegung oder eine Augen- oder Kopfbewegung ein Einschalten und Einstellen einer Röntgenröhre vornehmen. Die genannte Geste wird zum Beispiel von einer Bildaufnahmeeinheit erfasst und von einer an der medizintechnischen Bildgebungseinrichtung angeordneten festinstallierten Empfangseinheit empfangen und ausgewertet. Vorteilhaft können Steuerungsvorgänge durch eine Kontrollgeste mit Hilfe eines Gesten-Schuh-Sensors 20 zuverlässiger gemacht werden, so dass keine Steuerung aus Versehen erfolgen kann, die einem Patienten schaden könnte.

FIG 5 zeigt ein Computertomographiesystem 50 gemäß einem Ausführungsbeispiel der Erfindung. Der hier gezeigte Computertomograph verfügt über eine Aufnahmeeinheit 57, umfassend eine Strahlungsquelle 58 in Form einer Röntgenquelle sowie einen Strahlungsdetektor 59 in Form eines Röntgendetektors. Die Aufnahmeeinheit 57 rotiert während der Aufnahme von Röntgenprojektionen um eine Systemachse 55, und die Röntgenquelle 58 emittiert während der Aufnahme Strahlen 52 in Form von Röntgenstrahlen. Bei der Röntgenquelle 58 handelt es sich um eine Röntgenröhre. Bei dem Röntgendetektor 59 handelt es sich um einen Zeilendetektor mit mehreren Zeilen.

Ein Patient 53 liegt bei der Aufnahme von Projektionen auf einer Patientenliege 56. Die Patientenliege 56 ist so mit einem Liegensockel 54 verbunden, dass er die Patientenliege 56 mit dem Patienten 53 trägt. Die Patientenliege 56 ist dazu ausgelegt, den Patienten 53 entlang einer Aufnahmerichtung durch die Öffnung 60 der Aufnahmeeinheit 57 zu bewegen. Die Aufnahmerichtung ist in der Regel durch die Systemachse 55, welche in z-Richtung ausgerichtet ist, gegeben. Um die z-Achse rotiert die Aufnahmeeinheit 57 bei der Aufnahme von Röntgenprojektionen. In diesem Beispiel ist die Körperachse des Patienten gleich der Systemachse 55. Beide Achsen liegen auf der Z-Achse eines dreidimensionalen, kartesischen Koordinatensystems. Bei einer Spiral-Aufnahme wird die Patientenliege 56 kontinuierlich durch die Öffnung 60 bewegt, während die Aufnahmeeinheit 67 um den Patienten 53 rotiert und Röntgenprojektionen aufnimmt. Damit beschreiben die Röntgenstrahlen auf der Oberfläche des Patienten 53 eine Spirale.

Die Röntgenbildaufnahmevorrichtung 51 verfügt über einen Computer 62, welcher mit einer Anzeigeeinheit 61, beispielsweise zur graphischen Anzeige von Röntgenaufnahmen, sowie einer Eingabeeinheit 67 verbunden ist. Bei der Anzeigeeinheit 61 kann es sich beispielsweise um einen LCD-, Plasma- oder OLED-Bildschirm handeln. Es kann sich weiterhin um einen berührungsempfindlichen Bildschirm handeln, welcher auch als Eingabeeinheit 67 ausgebildet ist. Ein solcher berührungsempfindlicher Bildschirm kann in das bildgebende Gerät integriert oder als Teil eines mobilen Geräts ausgebildet sein. Bei der Eingabeeinheit 67 handelt es sich beispielsweise um eine Tastatur, eine Maus, einen sogenannten "Touch-Screen" oder auch um ein Mikrofon zur Spracheingabe. Die Eingabeeinheit 67 kann auch eingerichtet sein, um Bewegungen eines Benutzers zu erkennen und in entsprechende Befehle zu übersetzen. Mittels Eingabeeinheit 67 kann beispielsweise ein ausgewählter Referenz-Datensatz durch einen Benutzer modifiziert werden.

Der Computer 62 steht mit der drehbaren Aufnahmeeinheit 57 zum Datenaustausch in Verbindung. Über die Verbindung 64 werden einerseits Steuersignale für die Röntgenbildaufnahme vom Computer 62 an die Aufnahmeeinheit 57 übertragen, andererseits werden aufgenommene Projektions-Daten für eine Bildrekonstruktion an den Computer 62 übertragen. Die Verbindung 64 ist in bekannter Weise kabelgebunden oder kabellos realisiert.

Der Computer 62 weist eine Recheneinheit 66 auf. Die Recheneinheit 66 ist als Bild- bzw. Bilddatenbearbeitungseinheit ausgestaltet. Sie ist eingerichtet, im Bezug zu dem erfindungsgemäßen Verfahren stehende Datenverarbeitungsschritte durchzuführen. Die Recheneinheit 66 kann mit einem computerlesbaren Datenträger 63 zusammenwirken, insbesondere um durch ein Computerprogramm mit Programmcode Befehle entgegenzunehmen und zu verarbeiten und eine Bildgebung zu steuern. Weiterhin kann das Computerprogramm auf dem maschinenlesbaren Träger abrufbar gespeichert sein. Insbesondere kann es sich bei dem maschinenlesbaren Träger um eine CD, DVD, Blu-Ray Disc, einen Memory-Stick oder eine Festplatte handeln. Die Recheneinheit 66 kann in Form von Hard- oder in Form von Software ausgebildet sein. Beispielsweise ist die Recheneinheit 66 als ein sogenanntes FPGA (Akronym für das englischsprachige "Field Programmable Gate Array") ausgebildet oder umfasst eine arithmetische Logikeinheit.

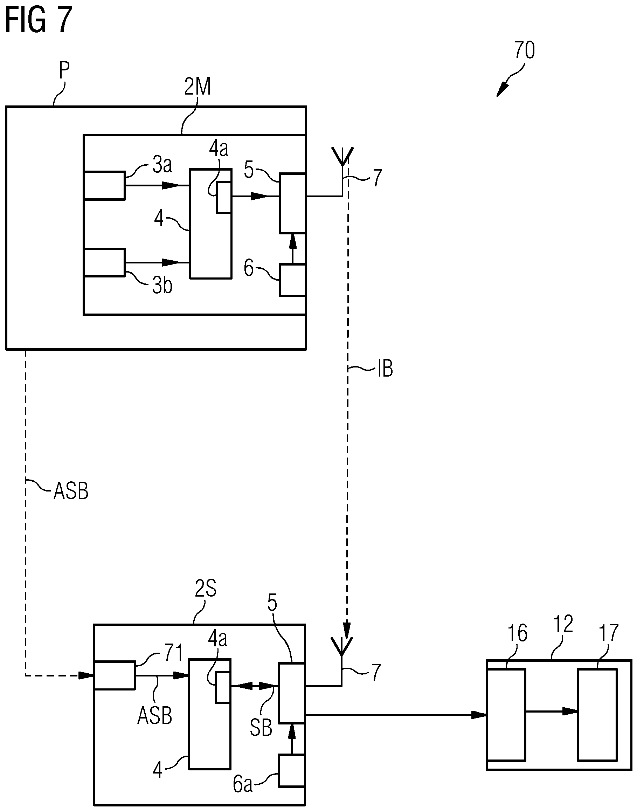

In FIG 5 ist auch ein Befehlsübermittlungssystem 70 (siehe im Detail in FIG 7) mit einer Sendeeinheit 2M und einer Empfangseinheit 2S gezeigt. Die Empfangseinheit 2S ist als Funkschnittstelle Teil des Computers 62 Slave-Schaltung ausgebildet. Die Sendeeinheit 2M ist in einen Schuh 1 einer Bedienperson P integriert und als Masterschaltung ausgeführt. Die Bedienperson P kann nun durch eine Bewegungssequenz des Schuhs 1 eine Spracherkennungsfunktion der als Slave-Schaltung ausgebildeten Empfangseinheit 2S freischalten. Anschließend gibt die Bedienperson P einen Sprachbefehl, der von der Empfangseinheit 2S empfangen und interpretiert wird. Auf Basis dieser Interpretation erzeugt die Empfangseinheit 2S einen Befehl zum Ausführen einer Funktion, wie zum Beispiel das Durchblättern von Bildern oder das Ändern eines Kontrasts. Zum Umsetzen des Befehls kann von der Empfangseinheit 2S auch ein als Aktor funktionierender Schalter 12 angesteuert werden. Der Schalter 12 dient zum Beispiel zum Auslösen der Strahlung durch die Strahlungsquelle 58.

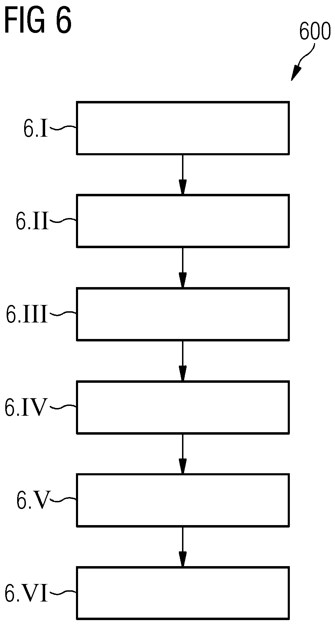

In FIG 6 ist ein Flussdiagramm 600 gezeigt, welches ein Befehlsübermittlungsverfahren gemäß einem Ausführungsbeispiel der Erfindung veranschaulicht. Bei dem Schritt 6.1 erfolgt ein Erfassen einer Bewegung einer Bedienperson P durch eine an der Bedienperson P angeordnete erste Befehlsübermittlungseinrichtung 2M. Beispielsweise steht ein erfahrener Radiologe, der gerade mit seinen Händen einen Patienten untersucht, neben einem mobilen C-Bogengerät. Der Radiologe hat also aufgrund der simultanen Untersuchung mit den Händen aktuell keine Hände frei, um das C-Bogengerät für eine Bildgebung von dem Patienten P zu betätigen. Daher trägt der Radiologe eine bereits in FIG 1 und FIG 2 beschriebene erste Befehlsübermittlungseinrichtung 2M in einem seiner Schuhe 1 oder beiden Schuhen 1. Die erste Befehlsübermittlungseinrichtung 2M ist in einem sogenannten Master-Modus initialisiert, d.h. in diesem Master-Modus wird nur der elektronische Sensor zur Bewegungserkennung aktiviert, die Spracherkennung ist aber deaktiviert, da die erste Befehlsübermittlungseinrichtung 2M in diesem Ausführungsbeispiel nur zum Freischalten einer Spracherkennung einer baugleichen zweiten Befehlsübermittlungseinrichtung 2M, welche an dem C-Bogengerät angeordnet ist, genutzt wird.

Weiterhin wird bei dem Schritt 6.II die erfasste Bewegung der Bedienperson, d.h. in diesem konkreten Ausführungsbeispiel des Radiologen, ausgewertet und es wird ein Befehl auf Basis der erfassten Bewegung identifiziert. Die Bewegung der Bedienperson P kann zum Beispiel eine Fußtippfolge von drei Fußtippbewegungen umfassen. In diesem konkreten Ausführungsbeispiel handelt es sich bei dem Befehl um einen Freischaltebefehl, mit dem die zweite Befehlsübermittlungseinrichtung 2S empfangsbereit für einen Sprachbefehl des Radiologen geschaltet wird. Bei dem Schritt 6.III wird dann der identifizierte Befehl drahtlos an die zweite Befehlsübermittlungseinrichtung 2S übermittelt, welche an der zu bedienenden medizintechnischen Einrichtung, in diesem Fall ein C-Bogengerät, angeordnet ist. Bei dem Schritt 6.IV erfolgt dann eine Aktivierung der Spracherkennung der zweiten Befehlsübermittlungseinrichtung 2S. Bei dem Schritt 6.V erfasst die zweite Befehlsübermittlungseinrichtung 2S einen von dem Radiologen gegebenen Sprachbefehl und wertet ihn durch die interne Spracherkennung aus. Beispielsweise wird das Wort "Umblättern" oder "Bild zurück" oder "Bild vor" erkannt. Dieser Befehl wird durch die zweite Befehlsübermittlungseinrichtung 2S als Befehl zum Umblättern zwischen mehreren Bilddatensätzen interpretiert, aber nur dann, wenn der bei dem Schritt 6.II beschriebene Gestenbefehl erfolgte und erkannt wurde, und es wird bei dem Schritt 6.VI ein entsprechender Steuerbefehl an eine Bilddarstellungseinheit eines C-Bogengeräts drahtlos oder per Kabel übermittelt, mit dem zwischen verschiedenen Bilddatensätzen hin- und hergewechselt wird. Durch diese zweistufige Vorgehensweise kann ein unbeabsichtigtes Auslösen einer Aktion vermieden werden.

In FIG 7 ist ein Befehlsübermittlungssystem 70 gemäß einem Ausführungsbeispiel der Erfindung schematisch gezeigt. Ein solches Befehlsübermittlungssystem 70 kommt zum Beispiel in dem in FIG 5 gezeigten System 50 zum Einsatz. Das in FIG 7 gezeigte Befehlsübermittlungssystem 70 umfasst eine an einer Kleidung einer Bedienperson P, beispielsweise einem Schuh, fixierte Sendeeinheit 2M und eine an einem bildgebenden medizintechnischen System (nicht gezeigt) montierte Empfangseinheit 2S. Der Aufbau der Sendeeinheit 2M entspricht weitgehend dem in FIG 2 gezeigten Aufbau der dort veranschaulichten Befehlsübermittlungseinrichtung 2. Ebenso wie die in FIG 2 gezeigte Befehlsübermittlungseinrichtung 2 weist auch die Sendeeinheit 2M eine Energieerzeugungs- und speichereinheit 6 in Form einer Energieernteeinheit auf, mit der die Bewegung der Bedienperson P in elektrische Energie gewandelt und diese auch gespeichert werden kann.

Zusätzlich umfasst die in FIG 7 gezeigte Sendeeinheit 2M eine Spracherkennungseinheit 4a auf, mit der akustische Sensorsignale, welche beispielsweise von einer externen akustischen Sensoreinheit erfasst wurden und über die Sende-Empfangseinheit 5 an die Spracherkennungseinheit 4a übermittelt werden, hinsichtlich inhaltlicher Informationen, d.h. auf Wörter und Satzteile hin untersucht werden können. Nun ist aber die Sendeeinheit 2M als Mastereinheit ausgebildet und dient der Initialisierung einer zweistufigen Befehlssequenz, mit der eine sicherheitskritische Ansteuerung eines medizintechnischen Geräts abgesichert vorgenommen werden kann. Daher wird die Spracherkennungseinheit 4a in der als Mastereinheit installierten Sendeeinheit 2M nicht benötigt und wird daher auch nicht freigeschaltet. Vorteilhaft kann aber ein weitgehend mit der Sendeeinheit 2S gleichartig aufgebautes Bauteil 2S als Empfangseinheit 2S genutzt werden, bei dem die beschriebene Funktion zur Auswertung akustischer Information und zur Spracherkennung zu Anwendung kommt. Die Empfangseinheit 2S unterscheidet sich von der in Sendeeinheit 2M dahingehend, dass ihre Energieversorgung 6a nicht auf Bewegungsverwertung beruht, da die Empfangseinheit 2S fest an einer medizintechnischen Einrichtung angeordnet ist und somit keine Bewegungsenergie zur Erzeugung elektrischer Energie nutzen kann. Die an der Empfangseinheit 2S montierte Energieversorgung 6a kann zum Beispiel eine Batterieeinheit oder einen Netzanschluss umfassen. Die Empfangseinheit 2S ist weiterhin mit einer Aktoreineinheit 12 über eine Datenübertragungsleitung verbunden. Die Aktoreinheit 12 umfasst einen System-Mikrokontroller 16 und zum Beispiel eine Schalteranordnung 17, mit der eine Funktion der medizintechnischen Einrichtung ausgelöst werden kann.

Die Funktionsweise des in FIG 7 gezeigten Befehlsübermittlungssystems 70 ist wie folgt: Möchte die Bedienperson P eine Funktion der medizintechnischen Einrichtung auslösen, so bewegt sie zunächst ihren Fuß auf eine vorbestimmte Weise. Beispielsweise tippt sie einmal mit der Ferse, einmal mit der Fußspitze und einmal erneut mit der Ferse. Die durch diese Bewegungen ausgelösten Sensorsignale der Drucksensoren 3a, 3b, 3c der Sendeeinheit 2M werden an die Auswertungseinheit 4 der Sendeeinheit 2M übermittelt. Die Auswertungseinheit 4 der Sendeeinheit 2M erkennt nun anhand der erfassten Sensorsignale, dass eine Freischaltung der Spracherkennung 4a der Empfangseinheit 2S erfolgen soll. Daher wird über die Funkeinheit 5 bzw. deren Antenne 7 ein Initialisierungsbefehl IB an die Funkeinheit 5 der Empfangseinheit 2S übermittelt. Die Empfangseinheit 2S wertet durch ihre Auswertungseinheit 4 den empfangenen Initialisierungsbefehl IB aus und schaltet ihre Spracherkennungseinheit 4a frei, weil sie nun als nächste einen Sprachbefehl ASB erwartet. Darauf gibt die Bedienperson P einen Sprachbefehl ASB, beispielsweise möchte sie mit den Worten "Energie ein" eine Strahlungsauslösung der medizintechnischen Einrichtung bewirken. Der Sprachbefehl ASB wird nun von einer akustischen Sensoreinheit 71, die Teil der Empfangseinheit 2M ist, beispielsweise ein Mikrofon, erfasst und an die Spracherkennungseinheit 4a der Auswertungseinheit 4 der Empfangseinheit 2S übermittelt. Die Spracherkennungseinheit 4a der Empfangseinheit 2S ermittelt nun den Inhalt des von der Bedienperson P gegebenen Sprachbefehls ASB. In diesem Fall wird ermittelt, dass eine Strahlungsauslösung umgesetzt werden soll. Also wird über die Datenübertragungseinheit 5 ein Steuerbefehl SB an die Aktoreinheit 12 übermittelt, eine Strahlungsauslösung vorzunehmen. Die sicherheitskritische Strahlungsauslösung wird also nur dann vorgenommen, wenn zwei unabhängige, auf unterschiedliche Weise erzeugte Befehle zeitlich kurz hintereinander gegeben werden, so dass ein Einschalten einer Bestrahlung aus Versehen vermieden werden kann.

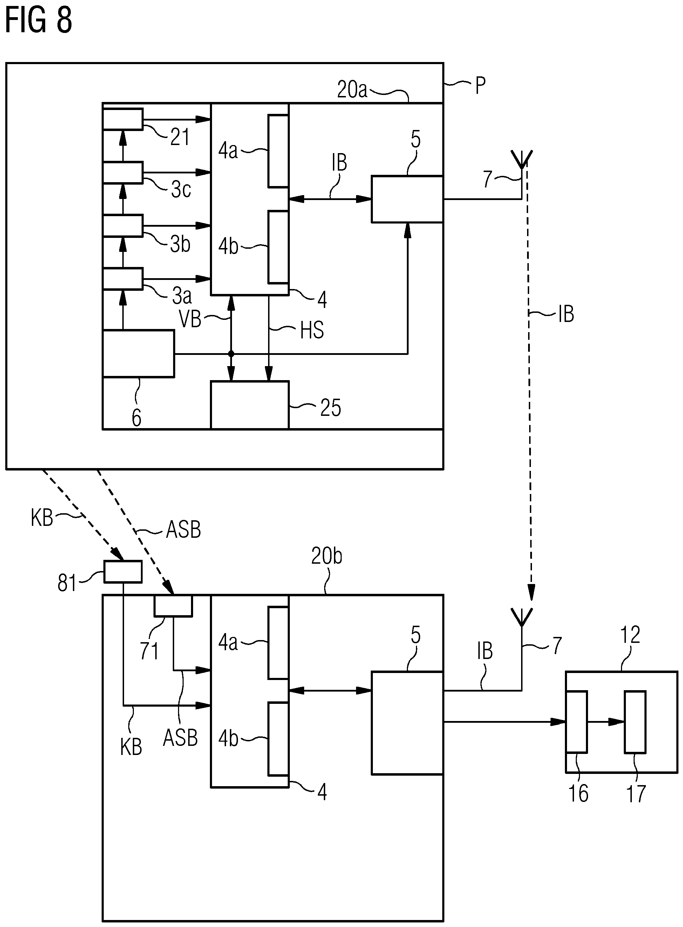

In FIG 8 ist ein Befehlsübermittlungssystem 80 gemäß einem alternativen Ausführungsbeispiel der Erfindung gezeigt. Das in FIG 8 gezeigte Befehlsübermittlungssystem 80 umfasst ebenfalls wie das in FIG 7 gezeigte Befehlsübermittlungssystem 70 eine Sendeeinheit 20a und eine Empfangseinheit 20b. Die Sendeeinheit 20a ist als Mastereinheit ausgebildet, d.h. die Sendeeinheit 20a dient dazu eine Befehlserfassung durch die als Slaveeinheit ausgebildete Empfangseinheit 20b zu kontrollieren.

Die bei dem in FIG 8 gezeigten Befehlsübermittlungssystem 80 genutzte Sendeeinheit 20a ist strukturell dem in FIG 4 gezeigten Gesten-Schuhsensor 20 nachgebildet. Insbesondere umfasst die in FIG 8 gezeigte Sendeeinheit 20a einen Inertialsensor 21, mit dem eine Geste, d.h. eine Bewegung eines Schuhs durch Beschleunigungsmessung und Messung einer Rotationsbewegung erfasst werden kann. Die Sendeinheit 20a umfasst eine Mehrzahl von unterschiedlichen Sensoren 3a, 3b, 3c, 21, um flexibel auf unterschiedliche Eingabearten reagieren zu können, sowie eine Auswertungseinheit 4 zur Auswertung von Sensorsignalen und zur Zuordnung von Befehlsinhalten zu erkannten Bewegungen, Sprachbefehlen oder optisch zu erkennenden Haltungen oder Gesten einer Bedienperson. Die Sendeeinheit 20a weist Drucksensoreinheiten 3a, 3b, 3c auf, mit denen eine Gewichtsverlagerung des Fußes, beispielsweise auf die Fußspitze oder die Fußferse erfasst werden kann. Zudem umfasst die Sendeeinheit 20a, wie bereits erwähnt, eine Inertialsensoreinheit 21, mit der beschleunigte Bewegungen eines Schuhs erfasst werden können. Weiterhin umfasst die Sendeeinheit 20a eine Spracherkennungseinheit 4a, die Teil der Auswertungseinheit 4 ist, mit der Sprachbefehle auf Basis von akustischen Messdaten erkannt werden können. Akustische Messdaten können zum Beispiel über die Sende-Empfangseinheit 5 von einem akustischen Sensor 71, beispielsweise ein Mikrofon, empfangen werden und von der Spracherkennungseinheit 4a ausgewertet werden. Anders als die bisher in FIG 1 bis FIG 7 gezeigten Varianten umfasst die in FIG 8 gezeigte Sendeeinheit 20a eine Bildanalyseeinheit 4b, die Teil der Auswertungseinheit 4 ist, zur Erkennung von Gesten der Bedienperson in Bilddaten, die beispielsweise von einer externen Videokamera 81 erfasst werden und anschließend durch die Bildanalyseeinheit 4b der Sendeeinheit 20a ausgewertet werden.

Außerdem umfasst die in FIG 8 gezeigte Sendeeinheit 20a ebenso wie der in FIG 4 gezeigte Schuhsensor 20 eine Einheit 25 zur Übermittlung eines haptischen Feedbacks. Bewegt eine Bedienperson P ihren Schuh oder führt sie eine anderweitig durch einen der beschriebenen Sensoren erfassbare Bewegung aus, um eine Gesten- oder Spracherkennungsfunktion der Empfangseinheit 20b freizuschalten, so werden die Sensorsignale von der Auswertungseinheit 4 der Sendeeinheit 20a ausgewertet und eine diesen Signalen zugeordnete Information ermittelt.

Die Auswertungseinheit 4 der Sendeeinheit 20a gibt für den Fall, dass sie eine Geste oder einen Sprachbefehl erkennt, die einen Initialisierungsbefehl IB kennzeichnen, ein entsprechendes Befehlssignal an die Sende-Empfangseinheit 5 aus und übermittelt zusätzlich ein Steuersignal HS an die Einheit 25 zur Übermittlung eines haptischen Feedbacks. Die Einheit 25 zur Übermittlung eines haptischen Feedbacks erzeugt daraufhin ein mechanisches Signal, beispielsweise eine Vibrationsbewegung VB, mit der die Bedienperson P bzw. der Träger des Gesten-Schuh-Sensors darüber informiert wird, dass der von ihm durch eine Geste und/oder einen Sprachbefehl erzeugte Befehl IB erkannt und weitergegeben wurde. Mit dem Befehl IB wird dann zum Beispiel eine Eingabe für eine medizintechnische Bildgebungseinrichtung freigeschaltet. Anschließend kann die Bedienperson P durch eine weitere Geste, beispielsweise durch eine Handbewegung oder eine Augen- oder Kopfbewegung, oder einen Sprachbefehl oder eine Kombination daraus, eine Funktion einer medizintechnischen Bildgebungseinrichtung auslösen.

Beispielsweise wird ein Initialisierungsbefehl IB ermittelt, mit dem eine visuelle Gestenerkennung 4b sowie eine Spracherkennung 4a der Auswertungseinheit 4 der Empfangseinheit 20b freigeschaltet werden sollen. Nachdem die Empfangseinheit 20b, welche im Wesentlichen gleichartig wie die Sendeeinheit 20a aufgebaut ist, den Initialisierungsbefehl IB über ihre Sende-Empfangseinheit 5 empfangen hat und ihre Spracherkennungseinheit 4a und ihre Bildanalyseeinheit 4b freigeschaltet hat, kann die Bedienperson P eine Geste, beispielsweise eine Kopfbewegung KB, ausführen und gleichzeitig einen Sprachbefehl ASB abgeben. Die Kopfbewegung KB wird von einer externen Kamera 81 erfasst und an die Sende-Empfangseinheit 5 der Empfangseinheit 20b übermittelt. Der Sprachbefehl wird von einem in die Empfangseinheit 20b integrierten Mikrofon erfasst und an die Spracherkennungseinheit 4a der Empfangseinheit 20b übermittelt.

Der Mikrokontroller 4 der Sendeeinheit 20a wertet auch Sensorsignale der Sensoren 3a, 3b, 3c aus und für den Fall, dass er eine Geste erkennt, die einen Initialisierungsbefehl IB kennzeichnet, gibt er ein entsprechendes Befehlssignal an die Sende-Empfangseinheit 5 aus und übermittelt zusätzlich ein Steuersignal HS an die Einheit 25 zur Übermittlung eines haptischen Feedbacks. Die Einheit 25 zur Übermittlung eines haptischen Feedbacks erzeugt daraufhin ein mechanisches Signal, beispielsweise eine Vibrationsbewegung VB, mit der die Bedienperson bzw. der Träger des Gesten-Schuh-Sensors 20a darüber informiert wird, dass der von ihm durch eine Geste erzeugte Befehl erkannt und weitergegeben wurde. Mit dem Befehl wird dann zum Beispiel eine Eingabe für eine medizintechnische Bildgebungseinrichtung freigeschaltet. Anschließend kann die Bedienperson durch eine weitere Geste, beispielsweise durch eine Handbewegung oder eine Augen- oder Kopfbewegung und möglicherweise einen simultanen Sprachbefehl ein Einschalten und Einstellen einer Röntgenröhre vornehmen.

Die Empfangseinheit 20b ist analog zu der Sendeeinheit 20a aufgebaut entbehrt allerdings einer Mehrzahl von unterschiedlichen Sensoren 3a, 3b, 3c, 21, da diese für die Empfangsfunktion nicht benötigt werden. Weiterhin umfasst die Empfangseinheit 20a eine Spracherkennungseinheit 4a, mit der beispielsweise Sprachbefehle von einem Mikrofon 71 empfangen bzw. erfasst und ausgewertet werden können. Analog zu der Sendeinheit 20a umfasst die in FIG 8 gezeigte Empfangseinheit 20b auch eine Bildanalyseeinheit 4b zur Auswertung von Bilddaten von einer externen optischen Sensoreinheit, beispielsweise eine Videokamera 81. Mit der Videokamera 81 können zum Beispiel Gesten der Bedienperson P erfasst und anschließend durch die Auswertungseinheit 4 bzw. die Bildanalyseeinheit 4b der Empfangseinheit 20b ausgewertet werden. Werden nun Gesten und Sprachbefehle von der Bildanalyseeinheit 4b bzw. der Spracherkennungseinheit 4a der Empfangseinheit 20b erkannt, so wird ein Aktionsbefehl an eine Aktoreinheit 12 weitergeben, die eine Funktion der medizintechnischen Bildgebungseinrichtung auslöst. Hierzu umfasst die Aktoreinheit einen System-Mikrokontroller 16 und zum Beispiel eine Schalteranordnung 17, mit der eine Funktion der medizintechnischen Einrichtung ausgelöst werden kann.

Es wird abschließend noch einmal darauf hingewiesen, dass es sich bei den vorbeschriebenen Verfahren und Vorrichtungen lediglich um bevorzugte Ausführungsbeispiele der Erfindung handelt und dass die Erfindung vom Fachmann variiert werden kann, ohne den Bereich der Erfindung zu verlassen, soweit er durch die Ansprüche vorgegeben ist. So wurden das Verfahren und die Befehlsübermittlungseinrichtung in erster Linie anhand eines Systems zur Aufnahme von medizinischen Bilddaten erläutert. Die Erfindung ist jedoch nicht auf eine Anwendung im medizinischen Bereich beschränkt, sondern die Erfindung kann auch grundsätzlich auf die Aufnahme von Bildern für andere Zwecke angewandt werden. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließt der Begriff "Einheit" nicht aus, dass diese aus mehreren Komponenten besteht, die gegebenenfalls auch räumlich verteilt sein können.

## Patentansprüche

1. Befehlsübermittlungssystem (70, 80), aufweisend:
- eine erste Befehlsübermittlungseinrichtung (2M, 20a), welche
- eine Sensoreinheit (3a, 3b, 3c, 21, 71, 81) zum Erfassen einer Bewegung einer Bedienperson (P),
- eine Fixierungseinheit (2a) zum Fixieren der Sensoreinheit (3a, 3b, 3c, 21, 71, 81) und/oder der Befehlsübermittlungseinrichtung an der Bedienperson (P),
- eine Auswertungseinheit (4, 4a, 4b, 15, 22) zum Auswerten der erfassten Bewegung der Bedienperson (P) und zum Identifizieren eines Befehls (IB, ASB, KB) auf Basis der erfassten Bewegung, und
- eine Befehlsübermittlungseinheit zum Übermitteln eines Steuerbefehls auf Basis des identifizierten Befehls (IB, ASB, KB) an eine zu bedienende medizintechnische Einrichtung (50) aufweist,
wobei die Sensoreinheit (3a, 3b, 3c, 21) eine interne Gestensensoreinheit für eine Gestenerkennung umfasst,
- als Mastereinrichtung für einen bidirektionalen Austausch zu mindestens einer Slave-Einrichtung ausgebildet ist,
- deren Gestensensoreinheit (3a, 3b, 3c, 21) aktiviert ist und
- dazu eingerichtet ist, einen ermittelten Befehl (IB) an eine zweite Befehlsübermittlungseinrichtung (2S, 20b) zu übermitteln,
- die zweite Befehlsübermittlungseinrichtung (2S, 20b), welche
- entfernt von der Bedienperson (P), vorzugsweise an einer zu bedienenden medizintechnischen Einrichtung (50) angeordnet ist,
- als Slave-Einrichtung für einen bidirektionalen Datenaustausch mit einer Master-Einrichtung ausgebildet ist,
- eine Auswertungseinheit (4) aufweist, die mindestens einer der folgenden Untereinheiten umfasst:
- eine Gestenerkennungseinheit (4b), die zur Erfassung einer Bewegung oder Geste einer Bedienperson (P) aus einer Distanz zu der Bedienperson (P) aktiviert ist,
- eine Spracherkennungseinheit (4a) mit einer Spracherkennungsfähigkeit, und
- dazu eingerichtet ist, den von der ersten Befehlsübermittlungseinrichtung (2M, 20a) empfangenen Befehl (IB) zu empfangen und basierend auf dem empfangenen Befehl (IB) einen Steuerbefehl (SB) an die medizintechnische Einrichtung (50) zu übermitteln.

2. Befehlsübermittlungssystem nach Anspruch 1, wobei die Gestenerkennung der ersten Befehlsübermittlungseinrichtung durch Erfassung einer Bewegung der Sensoreinheit (3a, 3b, 3c, 21) oder einer mechanischen Einwirkung auf die Sensoreinheit (3a) erfolgt.

3. Befehlsübermittlungssystem nach Anspruch 1 oder 2, wobei die Fixierungseinheit (2a) der ersten Befehlsübermittlungseinrichtung dazu ausgebildet ist, die Gestensensoreinheit (3a, 3b, 3c, 21) an oder in einem Kleidungsstück, vorzugsweise in einem Schuh (1), zu fixieren.

4. Befehlsübermittlungssystem nach Anspruch 3, wobei die Fixierungseinheit (2a) der ersten Befehlsübermittlungseinrichtung als Aussparung in der Sohle des Schuhs (1) ausgebildet ist.

5. Befehlsübermittlungssystem nach einem der vorstehenden Ansprüche, wobei die Befehlsübermittlungseinheit der ersten Befehlsübermittlungseinrichtung eine Funkübertragungseinheit zum Übermitteln des identifizierten Befehls (IB) an die zu bedienende medizintechnische Einrichtung (50) aufweist.

6. Befehlsübermittlungssystem nach einem der vorstehenden Ansprüche, wobei die erste Befehlsübermittlungseinrichtung ferner eine Energieerzeugungs- und speichereinheit (6) aufweist, welche vorzugsweise dazu eingerichtet ist, aus der Bewegungsenergie der Bedienperson (P) elektrische Energie zu erzeugen.

7. Befehlsübermittlungssystem nach einem der vorstehenden Ansprüche, wobei
- die Sensoreinheit (71) der ersten Befehlsübermittlungseinrichtung eine akustische Sensoreinheit umfasst und
- die ihre Auswertungseinheit (4, 15, 22) eine Spracherkennungseinheit (4a) zum Erkennen und Interpretieren von Sprachbefehlen der Bedienperson (P) umfasst.

8. Befehlsübermittlungssystem nach einem der vorherigen Ansprüche, wobei die interne Gestenerkennung der ersten Befehlsübermittlungseinrichtung eine der folgenden Funktionsbefehle erkennt:
- Scrollen von Bildern auf einer Bildanzeige eines medizintechnischen Geräts (50),
- Ändern von Parametern eines medizintechnischen Geräts (50).

9. Befehlsübermittlungssystem (2, 10, 20) nach einem der vorhergehenden Ansprüche, wobei
- die erste Befehlsübermittlungseinrichtung (2, 2M, 20a) dazu eingerichtet ist,
- eine Geste zu identifizieren, welche einen Sprachbefehl (ASB) initialisiert,
- auf Basis der identifizierten Geste eine Inbetriebnahme der zweiten Befehlsübermittlungseinrichtung (2S, 20b) zu initialisieren,
- die zweite Befehlsübermittlungseinrichtung (2S, 20b) dazu eingerichtet ist,
- einen Initialisierungsbefehl (IB) von der ersten Befehlsübermittlungseinrichtung (2M, 20a) zu empfangen,
- die Spracherkennungseinheit (4a) und/oder die Gestenerkennungseinheit (4b) auf Basis des Initialisierungsbefehls (IB) zu aktivieren.

10. Befehlsübermittlungssystem (70, 80) nach Anspruch9, wobei die zweite Befehlsübermittlungseinrichtung (2M, 20b) dazu eingerichtet ist, nach Aktivierung der Spracherkennungseinheit (4a) einen Sprachbefehl (ASB) von der Bedienperson (P) zu detektieren und zu identifizieren und auf Basis des identifizierten Sprachbefehls (ASB) einen Steuerbefehl (SB) an die medizintechnische Einrichtung (50) zu übermitteln.

11. Befehlsübermittlungssystem nach einem der vorhergehenden Ansprüche, wobei die Gestenerkennungseinheit (4b) oder die Spracherkennungseinheit (4a) eine Fähigkeit zur Erkennung folgender Bestätigungssignale umfasst:
- eine Strahlungsauslösung,
- Bewegungen des medizintechnischen Geräts (50).

12. Befehlsübermittlungssystem nach einem der vorgehenden Ansprüche, wobei die Gestenerkennungseinheit (4b) dazu ausgebildet ist, eine der folgenden Gesten zu erfassen:
- Hände oder Finger greifen, loslassen, zoomen, bewegen
- Augen bewegen sich nach links, rechts, oben, unten blinzeln,
- Kopfbewegung nach links oder rechts.

13. Befehlsübermittlungssystem nach einem der vorhergehenden Ansprüche, wobei die erste Befehlsübermittlungseinrichtung (2M, 20a) dazu eingerichtet ist, der Bedienperson (P) in Antwort auf einen erkannten Befehl eine haptische Rückmeldung zu übermitteln.

14. Operationssystem, aufweisend,
- eine bildgebende medizintechnische Einrichtung (50),
- eine Operationseinrichtung,
ein Befehlsübermittlungssystem (70, 80) nach einem der vorhergehenden Ansprüche, eingerichtet zur Übermittlung eines Steuerbefehls (SB) an die bildgebende medizintechnische Einrichtung (50).

15. Befehlsübermittlungsverfahren, aufweisend die Schritte:
- Erfassen einer Bewegung einer Bedienperson mittels einer Sensoreinheit einer ersten Befehlsübermittlungseinrichtung, wobei die Sensoreinheit und/oder die erste Befehlsübermittlungseinrichtung mittels einer Fixierungseinheit an der Bedienperson fixiert ist,
- Auswerten der erfassten Bewegung der Bedienperson und Identifizieren eines Befehls auf Basis der erfassten Bewegung mittels einer Auswertungseinheit der ersten Befehlsübermittlungseinrichtung,
- Übermitteln eines Steuerbefehls auf Basis des identifizierten Befehls an eine zu bedienende medizintechnische Einrichtung mittels einer Befehlsübermittlungseinheit der ersten Befehlsübermittlungseinrichtung,
wobei
∘ die Sensoreinheit eine interne und aktivierte Gestensensoreinheit für eine Gestenerkennung umfasst,
∘ die erste Befehlsübermittlungseinrichtung
• als Mastereinrichtung für einen bidirektionalen Austausch zu mindestens einer Slave-Einrichtung ausgebildet und dazu eingerichtet ist, einen ermittelten Befehl an eine zweite Befehlsübermittlungseinrichtung zu übermitteln,
∘ die zweite Befehlsübermittlungseinrichtung
• entfernt von der Bedienperson, vorzugsweise an der zu bedienenden medizintechnischen Einrichtung, angeordnet ist,
• als Slave-Einrichtung für einen bidirektionalen Datenaustausch mit der Master-Einrichtung ausgebildet ist,
• eine Auswertungseinheit aufweist, die mindestens eine der folgenden Untereinheiten umfasst:
▪ eine Gestenerkennungseinheit (4b), die zur Erfassung einer Bewegung oder Geste einer Bedienperson (P) aus einer Distanz zu der Bedienperson (P) aktiviert ist, und
▪ eine Spracherkennungseinheit (4a) mit einer Spracherkennungsfähigkeit,
und
- Empfangen mittels der zweiten Befehlsübermittlungseinrichtung des von der ersten Befehlsübermittlungseinrichtung übermittelten Befehls und
- Übermitteln basierend auf dem empfangenen Befehl eines Steuerbefehls an die medizinische Einrichtung.

16. Computerprogrammprodukt mit einem Computerprogramm, welches direkt in einer Speichereinrichtung eines Befehlsübermittlungssystems (70, 80) gespeichert ist, mit Programmabschnitten, um alle Schritte des Verfahrens nach Anspruch 15 auszuführen, wenn das Computerprogramm in dem Befehlsübermittlungssystem (70, 80) ausgeführt wird.

17. Computerlesbares Medium, auf welchem von einer Rechnereinheit einlesbare und ausführbare Programmabschnitte gespeichert sind, um alle Schritte des Verfahrens nach Anspruch 15 auszuführen, wenn die Programmabschnitte von der Rechnereinheit ausgeführt werden.

## Claims

1. Command communication system (70, 80), having:
- a first command communication facility (2M, 20a), which has
- a sensor unit (3a, 3b, 3c, 21, 71, 81) for detecting a movement of an operator (P),
- a fixing unit (2a) for securing the sensor unit (3a, 3b, 3c, 21, 71, 81) and/or the command communication facility to the operator (P),
- an evaluation unit (4, 4a, 4b, 15, 22) for evaluating the detected movement of the operator (P) and for identifying a command (IB, ASB, KB) on the basis of the detected movement, and
- a command communication unit for transmitting a control command to a medical facility (50) that is to be operated on the basis of the identified command (IB, ASB, KB),
wherein the sensor unit (3a, 3b, 3c, 21) comprises an internal gesture sensor unit for gesture recognition,
- is embodied as a master facility for a bidirectional exchange with at least one slave facility,
- whose gesture sensor unit (3a, 3b, 3c, 21) is activated
and
- which is configured to transmit a determined command (IB) to a second command communication facility (2S, 20b),
- the second command communication facility (2S, 20b), which
- is disposed at a distance from the operator (P), preferably on a medical facility (50) that is to be operated,
- is embodied as a slave facility for a bidirectional data exchange with a master facility,
- includes an evaluation unit (4) which comprises at least one of the following subunits:
- a gesture recognition unit (4b) which is activated from a distance to the operator (P) for the purpose of detecting a movement or gesture of an operator (P),
- a voice recognition unit (4a) having a voice recognition capability, and
- which is configured to receive the command (IB) received by the first command communication facility (2M, 20a) and to transmit a control command (SB) to the medical facility (50) based on the received command (IB).

2. Command communication system according to claim 1, wherein the gesture recognition of the first command communication facility is realized by detection of a movement of the sensor unit (3a, 3b, 3c, 21) or of a mechanical force acting on the sensor unit (3a).

3. Command communication system according to claim 1 or 2, wherein the fixing unit (2a) of the first command communication facility is embodied to secure the gesture sensor unit (3a, 3b, 3c, 21) to or in an article of clothing, preferably in a shoe (1).

4. Command communication system according to claim 3, wherein the fixing unit (2a) of the first command communication facility is embodied as a recess in the sole of the shoe (1).

5. Command communication system according to one of the preceding claims, wherein the command communication unit of the first command communication facility includes a radio transmission unit (5, 7) for transmitting the identified command (IB) to the medical facility (50) that is to be operated.

6. Command communication system according to one of the preceding claims, wherein the first command communication facility further has an energy generation and storage unit (6) which is preferably configured to generate electrical energy from the kinetic energy of the operator (P).

7. Command communication system according to one of the preceding claims, wherein
- the sensor unit (71) of the first command communication facility comprises an acoustic sensor unit and
- the evaluation unit (4, 15, 22) comprises a voice recognition unit (4a) for recognizing and interpreting voice commands of the operator (P).

8. Command communication system according to one of the preceding claims, wherein the internal gesture recognition function of the first command communication facility recognizes one of the following function commands:
- scrolling images on an image display of a medical device (50),
- changing parameters of a medical device (50).

9. Command communication system (2, 10, 20) according to one of the preceding claims, wherein
- the first command communication facility (2, 2M, 20a) is configured to
- identify a gesture which initializes a voice command (ASB),
- initialize a startup of the second command communication facility (2S, 20b) on the basis of the identified gesture,
- the second command communication facility (2S, 20b) is configured to
- receive an initialization command (IB) from the first command communication facility (2M, 20a),
- activate the voice recognition unit (4a) and/or the gesture recognition unit (4b) on the basis of the initialization command (IB).

10. Command communication system (70, 80) according to claim 9, wherein the second command communication facility (2M, 20b) is configured to detect and identify a voice command (ASB) from the operator (P) following activation of the voice recognition unit (4a) and to transmit a control command (SB) to the medical facility (50) on the basis of the identified voice command (ASB).

11. Command communication system according to one of the preceding claims, wherein the gesture recognition unit (4b) or the voice recognition unit (4a) comprises a capability to recognize the following confirmation signals:
- a release of radiation,
- movements of the medical device (50).

12. Command communication system according to one of the preceding claims, wherein the gesture recognition unit (4b) is embodied to detect one of the following gestures:
- gripping or releasing using hands or fingers, zooming, moving,
- moving eyes left, right, up, down, blinking,
- moving head left or right.

13. Command communication system according to one of the preceding claims, wherein the first command communication facility (2M, 20a) is configured to transmit haptic feedback to the operator (P) in response to a recognized command.

14. Operating system having,
- an imaging medical facility (50),
- an operating facility,
a command communication system (70, 80) according to one of the preceding claims, configured to transmit a control command (SB) to the imaging medical facility (50).

15. Command communication method having the steps:
- detecting a movement of an operator by means of a sensor unit of a first command communication facility,
wherein the sensor unit and/or the first command communication facility is secured to the operator by means of a fixing unit,
- evaluating the detected movement of the operator and identifying a command on the basis of the detected movement by means of an evaluation unit of the first command communication facility,
- transmitting a control command to a medical facility that is to be operated on the basis of the identified command by means of a command communication unit of the first command communication facility,
wherein
o the sensor unit comprises an internal and activated gesture sensor unit for gesture recognition,
o the first command communication facility
• is embodied as a master facility for a bidirectional exchange with at least one slave facility and is configured to transmit a determined command to a second command communication facility,
∘ the second command communication facility
• is disposed at a distance from the operator, preferably on a medical facility that is to be operated,
• is embodied as a slave facility for a bidirectional data exchange with a master facility,
• includes an evaluation unit which comprises at least one of the following subunits:
• a gesture recognition unit (4b) which is activated from a distance to the operator (P) for the purpose of detecting a movement or gesture of an operator (P), and
• a voice recognition unit (4a) with a voice recognition capability,
and
- receiving the command transmitted by the first command communication facility by means of the second command communication facility and
- transmitting a control command to the medical facility based on the command received.

16. Computer program product with a computer program which is stored directly in a memory facility of a command communication system (70, 80), with program sections for performing all the steps of the method according to claim 15 when the computer program is executed in the command communication system (70, 80).

17. Computer-readable medium on which program sections that can be read in and executed by a computer unit are stored in order to perform all the steps of the method according to claim 15 when the program sections are executed by the computer unit.

## Revendications

1. Système (70, 80) de transmission d'instructions, comportant
- un premier dispositif (2M, 20a) de transmission d'instructions, qui a
- une unité (3a, 3b, 3c, 21, 71, 81) de capteur pour la détection d'un mouvement d'une personne (P) de service,
- une unité (2a) de fixation pour la fixation de l'unité (3a, 3b, 3c, 21, 71, 81) de capteur et/ou du dispositif de transmission d'instructions sur la personne (P) de service,
- une unité (4, 4a, 4b, 15, 22) d'évaluation pour l'évaluation du mouvement détecté de la personne (P) de service et pour l'identification d'une instruction (IB, ASB, KB) sur la base du mouvement détecté, et
- une unité de transmission d'instructions pour la transmission d'une instruction de commande sur la base de l'instruction (IB, ASB, KB) identifiée à un dispositif (50) de la technique médicale à desservir,
dans lequel l'unité (3a, 3b, 3c, 21) de capteur comprend une unité interne de capteur de gestes pour une reconnaissance de gestes,
- est constitué en dispositif maître pour un échange bidirectionnel avec au moins un dispositif esclave,
- dont l'unité (3a, 3b, 3c, 21) de capteur de gestes est activée et
- est agencée pour transmettre une instruction (IB) déterminée à un deuxième dispositif (2S, 20b) de transmission d'instructions,
- le deuxième dispositif (2S, 20b) de transmission d'instructions, qui
- est monté en étant loin de la personne (P) de service, de préférence sur un dispositif (50) de la technique médicale à desservir,
- est constitué en dispositif esclave pour un échange de données bidirectionnel avec un dispositif maître,
- a une unité (4) d'évaluation, qui comprend au moins l'une des sous-unités suivantes :
- une unité (4b) de reconnaissance de gestes, qui est activée à distance d'une personne (P) de service pour la détection d'un mouvement ou d'un geste de la personne (P) de service,
- une unité (4a) de reconnaissance vocale ayant une aptitude à la reconnaissance vocale, et
- est agencé pour recevoir l'instruction (IB) reçue du premier dispositif (2M, 20a) de transmission d'instructions et pour transmettre, sur la base de l'instruction (IB) reçue, une instruction (SB) de commande au dispositif (50) de la technique médicale.

2. Système de transmission d'instructions suivant la revendication 1, dans lequel la reconnaissance de gestes du premier dispositif de transmission d'instructions s'effectue par détection d'un mouvement de l'unité (3a, 3b, 3c, 21) de capteur ou d'une action mécanique sur l'unité (3a) de capteur.

3. Système de transmission d'instructions suivant la revendication 1 ou 2, dans lequel l'unité (2a) de fixation du premier dispositif de transmission d'instructions est constituée pour fixer l'unité (3a, 3b, 3c, 21) de capteur de gestes sur ou dans une pièce de vêtement, de préférence dans une chaussure (1).

4. Système de transmission d'instructions suivant la revendication 3, dans lequel l'unité (2a) de fixation du premier dispositif de transmission d'instructions est constituée sous la forme d'un évidement dans la semelle de la chaussure (1).

5. Système de transmission d'instructions suivant l'une des revendications précédentes, dans lequel l'unité de transmission d'instructions du premier dispositif de transmission d'instructions a une unité de transfert radio pour la transmission de l'instruction (IB) identifiée au dispositif (50) de la technique médicale à desservir.

6. Système de transmission d'instructions suivant l'une des revendications précédentes, dans lequel le premier dispositif de transmission d'instructions a en outre une unité (6) de production et d'accumulation d'énergie, laquelle est agencée de préférence pour produire de l'énergie électrique à partir de l'énergie cinétique de la personne (P) de service.

7. Système de transmission d'instructions suivant l'une des revendications précédentes, dans lequel
- l'unité (71) de capteur du premier dispositif de transmission d'instructions comprend une unité de capteur acoustique et
- son unité (4, 15, 22) d'évaluation comprend une unité (4a) de reconnaissance vocale pour la reconnaissance et l'interprétation d'instructions vocales de la personne (P) de service.

8. Système de transmission d'instructions suivant l'une des revendications précédentes, dans lequel la reconnaissance interne de gestes du premier dispositif de transmission d'instructions reconnaît l'une des instructions fonctionnelles suivantes :
- défilement d'images sur un affichage d'image d'un appareil (50) de la technique médicale,
- modification de paramètres d'un appareil (50) de la technique médicale.

9. Système (2, 10, 20) de transmission d'instructions suivant l'une des revendications précédentes, dans lequel
- le premier dispositif (2, 2M, 20a) de transmission d'instructions est agencé
- pour identifier un geste qui initialise une instruction (ASB) vocale,
- sur la base du geste identifié, pour initialiser une mise en fonctionnement du deuxième dispositif (2S, 20b) de transmission d'instructions,
- le deuxième dispositif (2S, 20b) de transmission d'instructions est agencé
- pour recevoir une instruction (IB) d'initialisation du premier dispositif (2M, 20a) de transmission d'instructions,
- pour activer l'unité (4a) de reconnaissance vocale et/ou l'unité (4b) de reconnaissance de gestes sur la base de l'instruction (IB) d'initialisation.

10. Système (70, 80) de transmission d'instructions suivant la revendication 9, dans lequel le deuxième dispositif (2M, 20b) de transmission d'instructions est agencé pour détecter par la personne (P) de service et pour identifier, après activation de l'unité (4a) de reconnaissance vocale, une instruction (ASB) vocale et pour transmettre, sur la base de l'instruction (ASB) vocale identifiée, une instruction (SB) de commande au dispositif (50) de la technique médicale.

11. Système de transmission d'instructions suivant l'une des revendications précédentes, dans lequel l'unité (4b) de reconnaissance de gestes ou l'unité (4a) de reconnaissance vocale comprend une aptitude à reconnaître des signaux d'actionnement suivants :
- un déclenchement de rayonnement,
- des mouvements de l'appareil (50) de la technique médicale.

12. Système de transmission d'instructions suivant l'une des revendications précédentes, dans lequel l'unité (4b) de reconnaissance de gestes est constituée pour détecter l'un des gestes suivants :
- préhension, relâchement, zoom, déplacement de mains ou de doigts,
- clignotement des yeux se déplaçant vers la gauche, la droite, le haut, le bas,
- mouvement de tête vers la gauche ou vers la droite.

13. Système de transmission d'instructions suivant l'une des revendications précédentes, dans lequel le premier dispositif (2M, 20a) de transmission d'instructions est agencé pour transmettre un message en retour haptique à la personne (P) de service en réponse à une instruction détectée.

14. Système opérationnel, comportant,
- un dispositif (50) d'imagerie de la technique médicale,
- un dispositif opérationnel,
un système (70, 80) de transmission d'instructions suivant l'une des revendications précédentes, agencé pour la transmission d'une instruction (SB) de commande au dispositif (50) d'imagerie de la technique médicale.

15. Procédé de transmission d'instructions, comportant les stades :
- détection d'un mouvement d'une personne de service au moyen d'une unité de capteur d'un premier dispositif de transmission d'instructions, dans lequel l'unité de capteur et/ou le premier dispositif de transmission d'instructions est fixé à la personne de service au moyen d'une unité de fixation,
- évaluation, au moyen d'une unité d'évaluation du premier dispositif de transmission d'instructions, du mouvement détecté de la personne de service et identification d'une instruction sur la base du mouvement détecté,
- transmission à un dispositif de la technique médicale à desservir, au moyen d'une unité de transmission d'instructions du premier dispositif de transmission d'instructions, d'une instruction de commande sur la base de l'instruction identifiée,
dans lequel
∘ l'unité de capteur comprend une unité interne et activée de capteur de gestes pour une reconnaissance de gestes,
∘ le premier dispositif de transmission d'instructions
• est constitué en dispositif maître pour un échange bidirectionnel avec au moins un dispositif esclave et est agencé pour transmettre une instruction déterminée à un deuxième dispositif de transmission d'instructions,
∘ le deuxième dispositif de transmission d'instructions
• est monté à distance de la personne de service, de préférence sur un dispositif de la technique médicale à desservir,
• est constitué en dispositif esclave pour un échange de données bidirectionnel avec le dispositif maître,
• a une unité d'évaluation, qui comprend au moins l'une des sous-unités suivantes :
▪ une unité (4b) de reconnaissance de gestes, qui est activée à distance d'une personne (P) de service pour la détection d'un mouvement ou d'un geste de la personne (P) de service, et
▪ une unité (4a) de reconnaissance vocale ayant une aptitude à la reconnaissance vocale,
et
- réception au moyen du deuxième dispositif de transmission d'instructions de l'instruction transmise par le premier dispositif de transmission d'instructions et
- transmission, sur la base de l'instruction reçue, d'une instruction de commande au dispositif médical.

16. Produit de programme d'ordinateur comprenant un programme d'ordinateur, qui est mis en mémoire directement dans un dispositif de mémoire d'un système (70, 80) de transmission d'instructions, comprenant des parties de programme pour exécuter tous les stades du procédé suivant la revendication 15, lorsque le programme d'ordinateur est exécuté dans le système (70, 80) de transmission d'instructions.

17. Support déchiffrable par ordinateur, sur lequel sont mises en mémoire des parties de programme déchiffrables et exécutables par une unité informatique, afin d'exécuter tous les stades du procédé suivant la revendication 15, lorsque les parties de programme sont exécutées par l'unité informatique.
